# EUROPÄISCHE PATENTSCHRIFT

(11) **EP 0 984 914 B1**
(45) Veröffentlichungstag und Bekanntmachung des Hinweises auf die Patenterteilung: **19.03.2003**
(21) Anmeldenummer: 98924202.9
(22) Anmeldetag: 24.04.1998
(51) Int. Cl.: C07C 49/835, C07C 49/813, C07C 205/45, C07C 317/24, C07C 45/54, A01N 35/06

(54) **SUBSTITUIERTE 2-(3-ALKENYL-BENZOYL)-CYCLOHEXAN-1,3-DIONE**
SUBSTITUTED 2-(3-ALKENYL-BENZOYL)-CYCLOHEXANE-1,3-DIONES
2-(3-ALKENYLE-BENZYOLE)-CYCLOHEXANE-1,3-DIONES SUBSTITUEES

(30) Priorität: 07.05.1997 DE 19719380
(43) Veröffentlichungstag der Anmeldung: 15.03.2000
(73) Patentinhaber: BASF AKTIENGESELLSCHAFT, 67056 Ludwigshafen (DE)
(72) Erfinder: BAUMANN, Ernst, D-67373 Dudenhofen (DE); VON DEYN, Wolfgang, D-67435 Neustadt (DE); ENGEL, Stefan, D-65510 Idstein (DE); HILL, Regina, Luise, D-67346 Speyer (DE); KARDORFF, Uwe, D-68159 Mannheim (DE); MAYER, Guido, D-67433 Neustadt (DE); OTTEN, Martina, D-67069 Ludwigshafen (DE); RACK, Michael, D-69123 Heidelberg (DE); RHEINHEIMER, Joachim, D-67063 Ludwigshafen (DE); WITSCHEL, Matthias, D-67061 Ludwigshafen (DE); WESTPHALEN, Karl-Otto, D-67346 Speyer (DE); MISSLITZ, Ulf, D-67433 Neustadt (DE); WALTER, Helmut, D-67283 Obrigheim (DE)
(86) Internationale Anmeldenummer: EP9802447
(87) Internationale Veröffentlichungsnummer: WO98050337

(56) Entgegenhaltungen:
- EP-A- 0 319 075
- DE-A- 4 241 999
- US-A- 4 822 906
- US-A- 4 997 473

## Beschreibung

Die vorliegende Erfindung betrifft 2-(3-Alkenyl-benzoyl)-cyclohexan-1,3-dione der Formel I 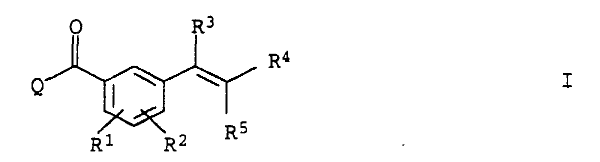 in der die Variablen folgende Bedeutung haben:
- R¹, R²: Wasserstoff, Nitro, Halogen, Cyano, Rhodano, C₁-C₆-Alkyl, C₁-C₆-Halogenalkyl, C₁-C₆-AlkoxyC₁-C₆-alkyl, C₂-C₆-Alkenyl, C₂-C₆-Alkinyl, -OR⁶, -OCOR⁷, -OSO₂R⁷, -SH, -S(O)ₙR⁸, -SO₂OR⁶, -SO₂NR⁶R⁹, -NR⁹SO₂R⁷ oder -NR⁹COR⁷;
- R³: Wasserstoff, Halogen, C₁-C₆-Alkyl, C₁-C₆-Halogenalkyl, C₁-C₆-Alkoxy, C₂-C₆-Alkenyl, C₂-C₆-Alkinyl;
- R⁴, R⁵: Wasserstoff, Nitro, Halogen, Cyano, Rhodano, C₁-C₆-Alkyl, C₁-C₆-Halogenalkyl, C₃-C₆-Cycloalkyl, C₂-C₆-Alkenyl, C₄-C₆-Cycloalkenyl, C₂-C₆-Alkinyl, C₁-C₆-Alkoxy, C₁-C₆-Alkylthio, C₁-C₆-Halogenalkoxy, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-Alkyl, das einen Rest aus folgender Gruppe trägt: Hydroxy, -COR¹⁰, -CO₂R¹⁰, -COSR¹⁰, -CONR¹⁰R¹¹ oder -C(R¹²)=NR¹³; Heterocyclyl, Heterocyclyl-C₁-C₄-alkyl, Phenyl, Phenyl-C₁-C₄-alkyl, Hetaryl, Hetaryl-C₁-C₄-alkyl, wobei die sechs letztgenannten Reste substituiert sein können;
oder
- R⁴ und R⁵: bilden gemeinsam eine C₂-C₆-Alkandiylkette, die einbis vierfach durch C₁-C₄-Alkyl substituiert sein kann und/oder durch Sauerstoff oder Schwefel oder einen gegebenenfalls C₁-C₄-Alkyl substituierten Stickstoff unterbrochen sein kann;
- n: 0, 1 oder 2;
- R⁶: Wasserstoff, C₁-C₆-Alkyl, C₁-C₆-Halogenalkyl, C₁-C₆-Alk- oxy-C₂-C₆-alkyl, C₃-C₆-Alkenyl oder C₃-C₆-Alkinyl;
- R⁷: C₁-C₆-Alkyl oder C₁-C₆-Halogenalkyl;
- R⁸: C₁-C₆-Alkyl, C₁-C₆-Halogenalkyl, C₁-C₆-Alkoxy-C₂-C₆alkyl, C₃-C₆-Alkenyl oder C₃-C₆-Alkinyl;
- R⁹: Wasserstoff oder C₁-C₆-Alkyl;
- R¹⁰: Wasserstoff, C₁-C₆-Alkyl, C₃-C₆-Cycloalkyl, C₁-C₆-Halogenalkyl, C₃-C₆-Alkenyl, C₃-C₆-Alkinyl, Phenyl oder Benzyl, wobei die beiden letztgenannten Reste partiell oder vollständig halogeniert sein können und/oder einen bis drei Reste aus der folgenden Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
- R¹¹: Wasserstoff, C₁-C₆-Alkyl, C₃-C₆-Alkenyl oder C₃-C₆-Alkinyl;
oder
- R¹⁰ und R¹¹: bilden gemeinsame eine C₂-C₆-Alkandiylkette, die einbis vierfach durch C₁-C₄-Alkyl substituiert sein kann und/oder durch Sauerstoff oder Schwefel oder einen gegebenenfalls C₁-C₄-Alkyl substituierten Stickstoff unterbrochen sein kann;
- R¹²: Wasserstoff, C₁-C₆-Alkyl, C₁-C₆-Halogenalkyl, C₁-C₆-Alkoxy, C₁-C₆-Alkoxycarbonyl, C₃-C₆-Cycloalkyl, C₂-C₆-Alkenyl, C₂-C₆-Alkinyl, Phenyl oder Benzyl, wobei die beiden letztgenannten Reste partiell oder vollständig halogeniert sein können und/oder einen bis drei Reste aus der folgenden Gruppe tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
- R¹³: C₁-C₆-Alkyl, C₁-C₆-Halogenalkyl, C₃-C₆-Cycloalkyl, C₃-C₆-Alkenyl, C₃-C₆-Alkinyl, C₁-C₆-Alkoxy, C₁-C₆-Halogenalkoxy, C₃-C₆-Cycloalkyloxy, C₃-C₆-Alkenyloxy, C₃-C₆-Alkinyloxy, Phenyl, Benzyl oder Phenyl-C₁-C₄-alkoxy, wobei die drei letztgenannten Reste partiell oder vollständig halogeniert sein können und/oder einen bis drei Reste aus der folgenden Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
- Q: ein unsubstituierter oder substituierter in 2-Stellung verknüpfter Cyclohexan-1,3-dionring der Formel II 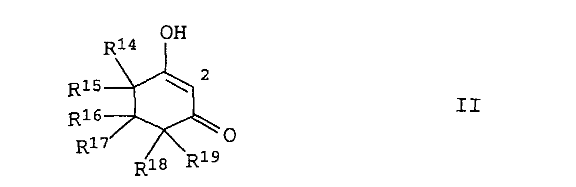 ist, wobei
R¹⁴, R¹⁵, R¹⁶, R¹⁷, R¹⁸ und R¹⁹ für Wasserstoff oder C₁-C₄-Alkyl stehen;
oder die CR¹⁶R¹⁷-Einheit durch C=O ersetzt sein kann;
sowie deren landwirtschaftlich brauchbaren Salze.

Außerdem betrifft die Erfindung Verfahren zur Herstellung von Verbindungen der Formel I, Mittel welche diese enthalten, sowie die Verwendung der Verbindungen der Formel I und diese enthaltende Mittel zur Schadpflanzenbekämpfung.

Aus WO 93/24446 sind in 2-Position substituierte Cyclohexan-1,3-dione bekannt, die anthelminthische und nematizide Eigenschaften besitzen. Weiterhin sind aus der Literatur, beispielsweise aus EP-A 135 191, EP-A 137 963 und EP-A 319 075 2-Benzoylcyclohexan-1,3-dione bekannt. Die herbiziden Eigenschaften dieser Verbindungen sowie die Verträglichkeiten gegenüber Kulturpflanzen können jedoch nur bedingt befriedigen. Es lag daher dieser Erfindung die Aufgabe zugrunde insbesondere herbizid wirksame Verbindungen mit verbesserten Eigenschaften zu finden.

Demgemäß wurden die 2-(3-Alkenyl-benzoyl)-cyclohexan-1,3-dione der Formel I sowie deren herbizide Wirkung gefunden.

Ferner wurden sehr gut wirksame herbizide Mittel gefunden, die die Verbindungen I enthalten. Außerdem wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon umfaßt.

Die Verbindungen der Formel I enthalten eine Kohlenstoff-Kohlenstoff-Doppelbindung und liegen daher als E-Isomere oder Z-Isomere oder als E/Z-Isomerengemische vor. Weiterhin können die Verbindungen der Formel I weitere Kohlenstoff-Kohlenstoff- bzw. Kohlenstoff-Stickstoff-Doppelbindungen enthalten. Gegenstand der Erfindung sind sowohl die reinen geometrischen Isomere als auch Gemische hiervon.

Die Verbindungen der Formel I können ebenso je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die Verbindungen der Formel I können ebenso auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-yl-ammonium, Di(2-hydroxyeth-1-yl)ammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹⁹ oder als Reste an Phenyl-, Hetaryl- und Heterocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Cycloalkoxy, Alkylthio, Alkylcarbonyl-, Alkoxycarbonyl-, Alkenyl-, Cycloalkenyl-, Alkinyl-, Alkenyloxy-, Alkinyloxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₂-C₄-Alkyl: Ethyl, n-Propyl, 1-Methylethyl, Butyl, 1-Methylpropyl, 2-Methylpropyl oder 1,1-Dimethylethyl;
- C₁-C₄-Alkyl, sowie die Alkylteile von C₁-C₄-Alkoxy-C₁-C₄alkyl, Hydroxy-C₁-C₄-alkoxy-C₁-C₄-alkyl, C₁-C₄-Alkylcarbonyl, Heterocyclyl-C₁-C₄-alkyl, Phenyl-C₁-C₄-alkyl, Hetaryl-C₁-C₄alkyl: C₂-C₄-Alkyl, wie voranstehend genannt sowie Methyl;
- C₂-C₆-Alkyl, sowie die Alkylteile von C₁-C₆-Alkoxy-C₂-C₆alkyl: C₂-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
- C₁-C₆-Alkyl, sowie die Alkylteile von C₁-C₆-Alkoxy-C₁-C₆alkyl: C₂-C₆-Alkyl, wie voranstehend genannt, sowie Methyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Halogenalkyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₄-Alkoxy, sowie die Alkoxyteile von C₁-C₄-Alkoxy-C₁-C₄alkyl, Hydroxy-C₁-C₄-alkoxy-C₁-C₄-alkyl, C₁-C₄-Alkoxycarbonyl: Methoxy, Ethoxy, Propoxy, 1-Methylethoxy, Butoxy, 1-Methylpropoxy, 2-Methylpropoxy oder 1,1-Dimethylethoxy;
- C₁-C₆-Alkoxy, sowie die Alkoxyteile von C₁-C₆-Alkoxy-C₁-C₆alkyl, und C₁-C₆-Alkoxycarbonyl: C₁-C₄-Alkoxy wie voranstehend genannt, sowie Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- C₁-C₆-Halogenalkoxy: C₁-C₄-Halogenalkoxy wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy;
- C₃-C₆-Cycloalkoxy: Cyclopropoxy, Cyclobutoxy, Cyclopentoxy oder Cyclohexoxy;
- C₁-C₄-Alkylthio: Methylthio, Ethylthio, Propylthio, 1-Methylethylthio, Butylthio, 1-Methylpropylthio, 2-Methylpropylthio oder 1,1-Dimethylethylthio;
- C₁-C₆-Alkylthio: C₁-C₄-Alkylthio wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₃-C₆-Alkenyl: Prop-1-en-1-yl, Prop-2-en-1-yl, 1-Methylethenyl, Buten-1-yl, Buten-2-yl, Buten-3-yl, 1-Methylprop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, l-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, l-Ethyl-1-methyl-prop-2-en-1-yl, 1-Ethyl-2-methylprop-1-en-1-yl oder 1-Ethyl-2-methyl-prop-2-en-1-yl;
- C₂-C₆-Alkenyl: C₃-C₆-Alkenyl wie voranstehend genannt, sowie Ethenyl;
- C₃-C₆-Alkinyl: Prop-1-in-1-yl, Prop-2-in-1-yl, But-1-in-1-yl, But-1-in-3-yl, But-1-in-4-yl But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl;
- C₂-C₆-Alkinyl: C₃-C₆-Alkinyl, wie voranstehend genannt, sowie Ethinyl:
- C₃-C₄-Cycloalkyl: Cyclopropyl oder Cyclobutyl;
- C₃-C₆-Cycloalkyl: C₃-C₄-Cycloalkyl, wie voranstehend genannt, sowie Cyclopentyl oder Cyclohexyl;
- C₃-C₆-Alkenyloxy: z.B. Prop-1-en-1-yloxy, Prop-2-en-1-yloxy, 1-Methylethenyloxy, Buten-1-yloxy, Buten-2-yloxy, Buten-3-yloxy, 1-Methyl-prop-1-en-1-yloxy, 2-Methyl-prop-1-en-1-yloxy, l-Methyl-prop-2-en-1-yloxy, 2-Methyl-prop-2-en-1-yloxy, Penten-1-yloxy, Penten-2-yloxy, Penten-3-yloxy, Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, l-Methyl-but-3-en-1-yloxy, 2-Methyl-but-3-en-1-yloxy, 3-Methyl-but-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethyl-prop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methylpent-2-en-1-yloxy, 1-Methyl-pent-3-en-1-yloxy, 2-Methylpent-3-en-1-yloxy, 3-Methyl-pent-3-en-1-yloxy, 4-Methylpent-3-en-1-yloxy, 1-Methyl-pent-4-en-1-yloxy, 2-Methylpent-4-en-1-yloxy, 3-Methyl-pent-4-en-1-yloxy, 4-Methylpent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, l-Ethyl-but-2-en-1-yloxy, 1-Ethyl-but-3-en-1-yloxy, 2-Ethylbut-1-en-1-yloxy, 2-Ethyl-but-2-en-1-yloxy, 2-Ethylbut-3-en-1-yloxy, 1,1,2-Trimethyl-prop-2-en-1-yloxy, 1-Ethyl-1-methyl-prop-2-en-1-yloxy, 1-Ethyl-2-methylprop-1-en-1-yloxy oder 1-Ethyl-2-methyl-prop-2-en-1-yloxy;
- C₃-C₆-Alkinyloxy: z.B. Prop-1-in-1-yloxy, Prop-2-in-1-yloxy, But-1-in-1-yloxy, But-1-in-3-yloxy, But-1-in-4-yloxy, But-2-in-1-yloxy, Pent-1-in-1-yloxy, Pent-1-in-3-yloxy, Pent-1-in-4-yloxy, Pent-1-in-5-yloxy, Pent-2-in-1-yloxy, Pent-2-in-4-yloxy, Pent-2-in-5-yloxy, 3-Methyl-but-1-in-3-yloxy, 3-Methyl-but-1-in-4-yloxy, Hex-1-in-1-yloxy, Hex-1-in-3-yloxy, Hex-1-in-4-yloxy, Hex-1-in-5-yloxy, Hex-1-in-6-yloxy, Hex-2-in-1-yloxy, Hex-2-in-4-yloxy, Hex-2-in-5-yloxy, Hex-2-in-6-yloxy, Hex-3-in-1-yloxy, Hex-3-in-2-yloxy, 3-Methylpent-1-in-1-yloxy, 3-Methylpent-1-in-3-yloxy, 3-Methyl-pent-1-in-4-yloxy, 3-Methylpent-1-in-5-yloxy 4-Methyl-pent-1-in-1-yloxy, 4-Methylpent-2-in-4-yloxy oder 4-Methylpent-2-in-5-yloxy;
- C₄-C₆-Cycloalkenyl: Cyclobuten-1-yl, Cyclobuten-3-yl, Cyclopenten-1-yl, Cyclopenten-3-yl, Cyclohexen-1-yl, Cyclohexen-3-yl oder Cyclohexen-4-yl;
- Heterocyclyl, sowie die Heteroclylreste in Heterocyclyloxy und Heterocyclyl-C₁-C₄-alkyl: drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 1,3-Dioxolan-4-yl, 1,3-Dithiolan-2-yl, 1,3-Dithiolan-4-yl, 1,3-Dioxan-2-yl, 1,3-Dioxan-4-yl, 1,3-Dioxan-5-yl, 1,3-Dithian-2-yl, 1,3-Dithian-4-yl, 1,3-Dithian-5-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl oder 1,3-Dihydrooxazin-2-yl;
- Hetaryl, sowie die Hetarylreste in Hetaryloxy und Hetaryl-C₁-C₄-alkyl:
   aromatische mono- oder polycyclische Reste, welche neben Kohlenstoffringgliedern zusätzlich ein bis vier Stickstoffatome oder ein bis drei Stickstoffatome und ein Sauerstoffoder ein Schwefelatom oder ein Sauerstoff- oder ein Schwefelatom enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl oder 1,2,4,5-Tetrazin-3-yl, sowie die entsprechenden benzokondensierten Derivate;
- C₂-C₆-Alkandiyl: z.B. Ethan-1,2-diyl, Propan-1,3-diyl, Butan-1,4-diyl, Pentan-1,5-diyl oder Hexan-1,6-diyl;

Alle Phenyl- und Hetarylringe sind vorzugsweise unsubstituiert oder tragen ein bis drei Halogenatome und/oder einen oder zwei Reste aus folgender Gruppe: Nitro, Cyano, Methyl, Trifluormethyl, Methoxy, Trifluormethoxy oder Methoxycarbonyl.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁶ oder -S(O)ₙR⁸; besonders bevorzugt Nitro, Halogen wie z.B. Fluor, Chlor oder Brom, C₁-C₆-Halogenalkyl, -OR⁶ oder -SO₂R⁸;
- R²: Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁶ oder -S(O)ₙR⁸;
besonders bevorzugt Wasserstoff, Nitro, Halogen wie z.B. Fluor, Chlor oder Brom, C₁-C₆-Alkyl, C₁-C₆-Halogenalkyl, -OR⁶ oder -SO₂R⁸;
insbesondere bevorzugt Nitro, Halogen wie z.B. Fluor, Chlor oder Brom, C₁-C₆-Alkyl wie z.B. Methyl, Ethyl, C₁-C₆-Halogenalkyl wie z.B. Difluormethyl, Trifluormethyl, -OR⁶ oder -SO₂R⁸;
- R³: Wasserstoff, Halogen, C₁-C₆-Alkyl, C₁-C₆-Halogenalkyl, C₁-C₆-Alkoxy, C₂-C₆-Alkenyl oder C₂-C₆-Alkinyl; besonders bevorzugt Wasserstoff, Halogen wie z.B. Fluor, Chlor oder Brom, C₁-C₄-Alkyl wie z.B. Methyl oder Ethyl, C₁-C₄-Halogenalkyl wie z.B. Trifluormethyl, C₁-C₄-Alkoxy wie z.B. Methoxy oder Ethoxy, Allyl oder Propargyl;
insbesondere bevorzugt Wasserstoff oder Methyl;
- R⁴: Wasserstoff, Nitro, Halogen, Cyano, Rhodano, C₁-C₆-Alkyl, C₁-C₆-Halogenalkyl, C₃-C₆-Cycloalkyl, C₂-C₆-Alkenyl, C₄-C₆-Cycloalkenyl, C₂-C₆-Alkinyl, C₁-C₆-Alkoxy, C₁-C₆-Alkylthio, C₁-C₆-Halogenalkoxy, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-Alkyl, das einen Rest aus folgender Gruppe trägt: -COR¹⁰, -CO₂R¹⁰, -COSR¹⁰, -CONR¹⁰R¹¹ oder -C(R¹²)=NR¹³; Heterocyclyl, Heterocyclyl-C₁-C₄-alkyl, Phenyl, Phenyl-C₁-C₄-alkyl, Hetaryl, Hetaryl-C₁-C₄-alkyl, wobei die sechs letztgenannten Reste ihrerseits substituiert sein können durch ein bis drei Halogenatome und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl, C₁-C₄-Alkoxycarbonyl;
- R⁵: Wasserstoff, Nitro, Halogen, Cyano, Rhodano, C₁-C₆-Alkyl, C₁-C₆-Halogenalkyl, C₃-C₆-Cycloalkyl, C₂-C₆-Alkenyl, C₄-C₆-Cycloalkenyl, C₂-C₆-Alkinyl, C₁-C₆-Alkoxy, C₁-C₆-Alkylthio, C₁-C₆-Halogenalkoxy, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-Alkyl, das einen Rest aus folgender Gruppe trägt: -COR¹⁰, -CO₂R¹⁰, -COSR¹⁰, -CONR¹⁰R¹¹ oder -C(R¹²)=NR¹³; Heterocyclyl, Heterocyclyl-C₁-C₄-alkyl, Phenyl, Phenyl-C₁-C₄-alkyl, Hetaryl, Hetaryl-C₁-C₄-alkyl, wobei die sechs letztgenannten Reste ihrerseits substituiert sein können durch ein bis drei Halogenatome und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl, C₁-C₄-Alkoxycarbonyl;
besonders bevorzugt Wasserstoff, Halogen, Cyano, Rhodano, C₁-C₆-Alkyl, C₁-C₆-Halogenalkyl, -COR¹⁰, -CO₂R¹⁰, -COSR¹⁰, -CONR¹⁰R¹¹ oder -PO(OR¹⁰)(OR¹¹);
oder
- R⁴ und R⁵: bilden gemeinsam eine C₂-C₆-Alkandiylkette, die ein bis vierfach durch C₁-C₄-Alkyl substituiert sein kann und/oder durch Sauerstoff oder Schwefel oder einen gegebenenfalls C₁-C₄-Alkyl substituierten Stickstoff unterbrochen sein kann beispielsweise Butan-1,4-diyl, Pentan-1,5-diyl, Hexan-1,6-diyl, 3-Oxapentan-1,5-diyl oder 3-Methyl-3-azapentan-1,5-diyl;
- n: 0 oder 2
- R⁶: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
besonders bevorzugt C₁-C₄-Alkyl wie z.B. Methyl oder Ethyl, C₁-C₄-Halogenalkyl wie z.B. Trifluormethyl oder Difluormethyl, C₁-C₄-Alkoxy-C₁-C₄-alkyl wie z.B. Methoxyethyl, Allyl oder Propargyl;
- R⁸: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl; besonders bevorzugt C₁-C₄-Alkyl wie z.B. Methyl oder Ethyl, C₁-C₄-Halogenalkyl wie z.B. Trifluormethyl, Difluormethyl, C₁-C₄-Alkoxy-C₁-C₄-alkyl wie z.B. Methoxyethyl, Allyl oder Propargyl;
- R¹⁰: Wasserstoff, C₁-C₆-Alkyl, C₃-C₆-Cycloalkyl, C₁-C₆-Halogenalkyl, C₃-C₆-Alkenyl, C₃-C₆-Alkinyl, Phenyl oder Benzyl; besonders bevorzugt Wasserstoff, C₁-C₄-Alkyl wie z.B. Methyl oder Ethyl, C₁-C₄-Halogenalkyl wie z.B. Trifluormethyl, Allyl, Propargyl und Benzyl;
- R¹¹: Wasserstoff, C₁-C₆-Alkyl, C₃-C₆-Alkenyl oder C₃-C₆-Alkinyl;
oder
- R¹⁰ und R¹¹: bilden gemeinsam eine C₂-C₆-Alkandiylkette, die ein-bis vierfach durch C₁-C₄-Alkyl substituiert sein kann;
- R¹²: Wasserstoff, C₁-C₆-Alkyl, C₁-C₆-Halogenalkyl, C₁-C₆-Alkoxy, C₁-C₆-Alkoxycarbonyl, C₃-C₆-Cycloalkyl, C₂-C₆-Alkenyl, C₂-C₆-Alkinyl, Phenyl oder Benzyl; besonders bevorzugt Wasserstoff, C₁-C₄-Alkyl wie z.B. Methyl oder Ethyl, C₁-C₄-Alkoxycarbonyl wie z.B. Methoxycarbonyl oder Ethoxycarbonyl, C₁-C₄-Halogenalkyl wie z.B. Trifluormethyl oder C₁-C₄-Alkoxy wie z.B. Methoxy oder Ethoxy;
- R¹³: C₁-C₆-Alkyl, C₁-C₆-Halogenalkyl, C₃-C₆-Cycloalkyl, C₃-C₆-Alkenyl, C₃-C₆-Alkinyl, C₁-C₆-Alkoxy, C₁-C₆-Halogenalkoxy, C₃-C₆-Cycloalkyloxy, C₃-C₆-Alkenyloxy, C₃-C₆-Alkinyloxy, Phenyl, Benzyl, oder Benzyloxy; besonders bevorzugt C₁-C₄-Alkyl wie z.B. Methyl oder Ethyl, C₁-C₄-Alkoxy wie z.B. Methoxy oder Ethoxy, Allyloxy, Propargyloxy, Benzyl oder Benzyloxy;
- R¹⁴, R¹⁵,: R¹⁷, R¹⁹ Wasserstoff oder C₁-C₄-Alkyl;
besonders bevorzugt Wasserstoff, Methyl oder Ethyl;
- R¹⁶: Wasserstoff oder C₁-C₄-Alkyl;
besonders bevorzugt Wasserstoff, Methyl oder Ethyl;
- R¹⁸: Wasserstoff oder C₁-C₄-Alkyl; besonders bevorzugt Wasserstoff oder Methyl.

Die CR¹⁶R¹⁷-Einheit kann auch vorteilhaft durch C=O ersetzt werden.

Außerordentlich bevorzugt sind Verbindungen der Formel I, wobei
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁶ oder -S(O)ₙR⁸;
- R²: Wasserstoff oder einen unter R¹, wie vorstehend, genannten Rest;
bedeutet.

Besonders außerordentlich bevorzugt sind Verbindungen der Formel I, wobei
- R¹: Nitro, Halogen wie z.B. Fluor, Chlor oder Brom, C₁-C₆-Halogenalkyl, OR⁶ oder SO₂R⁸;
- R²: Nitro, Halogen wie z.B. Fluor, Chlor oder Brom, C₁-C₆-Alkyl wie z.B. Methyl oder Ethyl, C₁-C₆-Halogenalkyl, OR⁶ oder SO₂R⁸ wie z.B. Methylsulfonyl oder Ethylsulfonyl;
bedeuten.

Ebenso außerordentlich bevorzugt sind Verbindungen der Formel I, wobei
- R⁴: Wasserstoff, Nitro, Halogen, Cyano, Rhodano, C₁-C₆-Alkyl, C₁-C₆-Halogenalkyl, C₁-C₆-Cycloalkyl, C₂-C₆-Alkenyl, C₄-C₆-Cycloalkenyl, C₂-C₆-Alkinyl, C₁-C₆-Alkoxy, C₁-C₆-Alkylthio, C₁-C₆-Halogenalkoxy, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-Alkyl, das einen Rest aus folgender Gruppe trägt: -COR¹⁰, -CO₂R¹⁰, -COSR¹⁰, -CONR¹⁰R¹¹ oder -C(R¹²)=NR¹³; Heterocyclyl, Heterocyclyl-C₁-C₄-alkyl, Phenyl, Phenyl-C₁-C₄-alkyl, Hetaryl, Hetaryl-C₁-C₄-alkyl, wobei die sechs letztgenannten Reste substituiert sein können;
- R⁵: Wasserstoff, Halogen, Cyano, Rhodano, C₁-C₆-Alkyl, C₁-C₆-Halogenalkyl, -COR¹⁰, -CO₂R¹⁰, -COSR¹⁰, -CONR¹⁰R¹¹ oder -PO(OR¹⁰) (OR¹¹);
oder
- R⁴ und R⁵: bilden gemeinsam eine C₂-C₆-Alkandiylkette, die ein-bis vierfach durch C₁-C₄-Alkyl substituiert sein kann und/oder durch Sauerstoff oder Schwefel oder einen gegebenenfalls C₁-C₄-Alkyl substituierten Stickstoff unterbrochen sein kann;
bedeutet.

Ebenso außerordentlich bevorzugt sind Verbindungen der Formel I, wobei
- R⁴: Nitro, Halogen, Cyano, Rhodano, C₁-C₆-Halogenalkyl, C₃-C₆-Cycloalkyl, C₂-C₆-Alkenyl, C₄-C₆-Cycloalkenyl, C₂-C₆-Alkinyl, C₁-C₆-Alkoxy, C₁-C₆-Alkylthio, C₁-C₆-Halogenalkoxy, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-Alkyl, das einen Rest aus folgender Gruppe trägt: -COR¹⁰, -CO₂R¹⁰, -COSR¹⁰, -CONR¹⁰R¹¹ oder -C(R¹²)=NR¹³; Heterocyclyl, Heterocyclyl-C₁-C₄-alkyl, Phenyl, Phenyl-C₁-C₄-alkyl, Hetaryl, Hetaryl-C₁-C₄-alkyl, wobei die sechs letztgenannten Reste substituiert sein können.
- R⁵: Wasserstoff, Halogen, Cyano, C₁-C₆-Alkyl, C₁-C₆-Halogenalkyl, -COR¹⁰, -CO₂R¹⁰, -COSR¹⁰, -CONR¹⁰R¹¹ oder -PO(OR¹⁰)(OR¹¹);
oder
- R⁴ und R⁵: bilden gemeinsam eine C₂-C₆-Alkandiylkette, die ein--bis vierfach durch C₁-C₄-Alkyl substituiert sein kann und/oder durch Sauerstoff oder Schwefel oder einen gegebenenfalls C₁-C₄-Alkyl substituierten Stickstoff unterbrochen sein kann;

Ebenso besonders bevorzugt sind Verbindungen der Formel I, wobei
- R¹: Nitro, Halogen, C₁-C₆-Halogenalkyl oder C₁-C₆-Alkylsulfonyl; insbesondere Nitro, Chlor, Trifluormethyl, Methylsulfonyl oder Ethylsulfonyl;
- R²: Nitro, Halogen, C₁-C₆-Alkyl, C₁-C₆-Halogenalkyl, C₁-C₆-Alkoxy oder C₁-C₆-Alkylsulfonyl;
insbesondere Nitro, Chlor, Methyl, Trifluormethyl, Methoxy oder Methylsulfonyl;
- R³: Wasserstoff oder C₁-C₆-Alkyl;
insbesondere Wasserstoff oder Methyl;
bevorzugt Wasserstoff;
- R⁴: Wasserstoff, Halogen, Cyano, C₁-C₄-Alkyl, C₁-C₄-Hydroxyalkyl, C₁-C₄-Alkoxy, C₁-C₄-Alkylcarbonyl, C₁-C₄-Alkoxycarbonyl oder -C(R¹²)=NR¹³;
insbesondere Wasserstoff, Chlor, Brom, Cyano, Methyl, Ethyl, 2-Hydroxyethyl, Methoxy, Ethoxy, Methylcarbonyl, Ethylcarbonyl, Methoxycarbonyl, Ethoxycarbonyl oder -C(R¹²)=NR¹³;
- R⁵: Wasserstoff, Halogen, C₁-C₄-Alkyl oder C₁-C₄-Alkoxy; insbesondere Wasserstoff, Halogen, Methyl, Ethyl, Methoxy oder Ethoxy;
- R¹²: Wasserstoff, C₁-C₄-Alkyl oder C₁-C₄-Alkoxy; insbesondere Wasserstoff oder Methyl;
- R¹³: C₁-C₆-Alkyl oder C₁-C₆-Alkoxy;
insbesondere Methoxy oder Ethoxy;
- R¹⁴, R¹⁵,: R¹⁶, R¹⁷, R¹⁸, R¹⁹ Wasserstoff oder C₁-C₄-Alkyl; insbesondere Wasserstoff oder Methyl;
bedeuten;
oder die CR¹⁶R¹⁷-Einheit durch C=O ersetzt sein kann; und wobei die Restedefinitionen R¹ bis R¹⁹ nicht nur in Kombination miteinander, sondern auch, jeweils für sich allein betrachtet, für die erfindungsgemäßen Verbindungen der Formel I eine besondere Bedeutung haben.

Besonders außerordentlich bevorzugt sind Verbindungen der Formel Ia (≙ I wobei R¹ in Position 4 des Phenylringes und R² in Position 2 des Phenylringes gebunden sind).

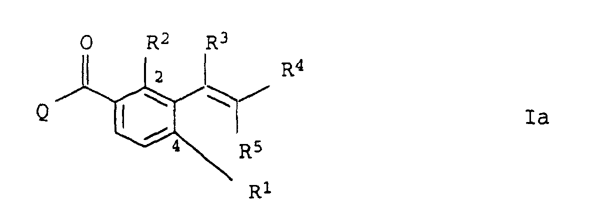

Insbesondere außerordentlich bevorzugt sind die Verbindungen Ia1 (≙ I mit R¹ = Cl, R¹⁴, R¹⁵, R¹⁶, R¹⁷, R¹⁸, R¹⁹ = H, wobei R¹ in 4-Position des Phenylringes und R² in 2-Position des Phenylringes gebunden sind), insbesondere die Verbindungen der Tabelle 1, wobei die Restedefinitionen R² bis R⁵ nicht nur in Kombination miteinander, sondern auch, jeweils für sich allein betrachtet, für die erfindungsgemäßen Verbindungen eine besondere Bedeutung haben.

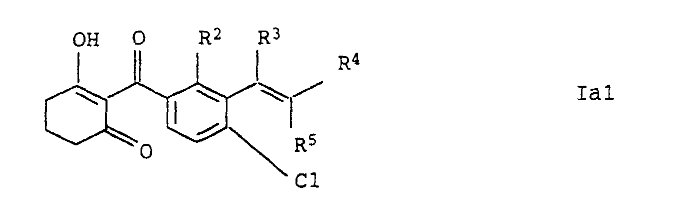

**Tabelle 1**

| Nr. | R² | R³ | R⁴ | R⁵ |
|---|---|---|---|---|
| Ia1.001 | Cl | H | H | H |
| Ia1.002 | Cl | H | CH₃ | H |
| Ia1.003 | Cl | H | CH₃ | CH₃ |
| Ia1.004 | Cl | H | C₂H₅ | H |
| Ia1.005 | Cl | H | n-C₃H₇ | H |
| Ia1.006 | Cl | H | n-C₄H₉ | H |
| Ia1.007 | Cl | H | CH(CH₃)₂ | H |
| Ia1.008 | Cl | H | cyclo-C₃H₅ | H |
| Ia1.009 | Cl | H | cyclo-C₄H₇ | H |
| Ia1.010 | Cl | H | cyclo-C₅H₉ | H |
| Ia1.011 | Cl | H | cyclo-C₆H₁₁ | H |
| Ia1.012 | Cl | H | C₆H₅ | H |
| Ia1.013 | Cl | H | CH₂-C₆H₅ | H |
| Ia1.014 | Cl | H | 2-Furyl | H |
| Ia1.015 | Cl | H | 3-Furyl | H |
| Ia1.016 | Cl | H | 2-Thienyl | H |
| Ia1.017 | Cl | H | 3-Thienyl | H |
| Ia1.018 | Cl | H | 1,3-Dioxan-2-yl | H |
| Ia1.019 | Cl | H | CHO | H |
| Ia1.020 | Cl | H | COCH₃ | H |
| Ia1.021 | Cl | H | COOCH₃ | H |
| Ia1.022 | Cl | H | COOC₂H₅ | H |
| Ia1.023 | Cl | H | OCH₃ | H |
| Ia1.024 | Cl | H | CN | H |
| Ia1.025 | Cl | H | SCH₃ | H |
| Ia1.026 | Cl | H | COCF₃ | H |
| Ia1.027 | Cl | H | COC₆H₅ | H |
| Ia1.028 | Cl | H | CH=NOCH₃ | H |
| Ia1.029 | Cl | H | CH=NOC₂H₅ | H |
| Ia1.030 | Cl | H | C(CH₃)=NOCH₃ | H |
| Ia1.031 | CH₃ | H | H | H |
| Ia1.032 | CH₃ | H | CH₃ | H |
| Ia1.033 | CH₃ | H | CH₃ | CH₃ |
| Ia1.034 | CH₃ | H | C₂H₅ | H |
| Ia1.035 | CH₃ | H | n-C₃H₇ | H |
| Ia1.036 | CH₃ | H | n-C₄H₉ | H |
| Ia1.037 | CH₃ | H | CHO | H |
| Ia1.038 | CH₃ | H | COCH₃ | H |
| Ia1.039 | CH₃ | H | COOCH₃ | H |
| Ia1.040 | CH₃ | H | OCH₃ | H |
| Ia1.041 | CH₃ | H | C₆H₅ | H |
| Ia1.042 | CH₃ | H | C₆H₅ | CH₃ |
| Ia1.043 | CH₃ | H | C₆H₅ | C₂H₅ |
| Ia1.044 | CH₃ | H | CH₂-CHO | H |
| Ia1.045 | CH₃ | H | COOCH₂C₆H₅ | H |
| Ia1.046 | CH₃ | Cl | CH₃ | H |
| Ia1.047 | CH₃ | CH₃ | CH₃ | H |
| Ia1.048 | CH₃ | C₂H₅ | CH₃ | H |
| Ia1.049 | CH₃ | CF₃ | CH₃ | H |
| Ia1.050 | CH₃ | OCH₃ | CH₃ | H |
| Ia1.051 | CH₃ | OC₂H₅ | CH₃ | H |
| Ia1.052 | CH₃ | CH₂-C≡CH | CH₃ | H |
| Ia1.053 | CH₃ | CH₂-CH=CH₂ | CH₃ | H |
| Ia1.054 | CH₃ | Cl | C₂H₅ | H |
| Ia1.055 | CH₃ | CH₃ | C₂H₅ | H |
| Ia1.056 | CH₃ | CF₃ | C₂H₅ | H |
| Ia1.057 | CH₃ | OCH₃ | C₂H₅ | H |
| Ia1.058 | CH₃ | OC₂H₅ | C₂H₅ | H |
| Ia1.059 | CH₃ | CH₂-C≡CH | C₂H₅ | H |
| Ia1.060 | CH₃ | CH₂-CH=CH | C₂H₅ | H |
| Ia1.061 | OCH₃ | H | H | H |
| Ia1.062 | OCH₃ | H | CH₃ | H |
| Ia1.063 | OCH₃ | H | C₂H₅ | H |
| Ia1.064 | OCH₃ | H | n-C₃H₇ | H |
| Ia1.065 | OCH₃ | H | n-C₄H₉ | H |
| Ia1.066 | OCH₃ | H | CHO | H |
| Ia1.067 | OCH₃ | H | COCH₃ | H |
| Ia1.068 | OCH₃ | H | COOCH₃ | H |
| Ia1.069 | OCH₃ | H | OCH₃ | H |
| Ia1.070 | OCH₃ | H | C₆H₅ | H |
| Ia1.071 | OCH₃ | H | CH=NOCH₃ | H |
| Ia1.072 | OCH₃ | H | C(CH₃)=NOCH₃ | H |
| Ia1.073 | OCH₃ | CH₃ | 2-Cl-C₆H₄ | H |
| Ia1.074 | OCH₃ | CH₃ | 3-Br-C₆H₄ | H |
| Ia1.075 | OCH₃ | CH₃ | 4-F-C₆H₄ | H |
| Ia1.076 | OCH₃ | CH₃ | 2,4-Cl₂-C₆H₃ | H |
| Ia1.077 | OCH₃ | CH₃ | 2-NO₂-C₆H₄ | H |
| Ia1.078 | OCH₃ | CH₃ | 3-CN-C₆H₄ | H |
| Ia1.079 | OCH₃ | CH₃ | 4-CH₃-C₆H₄ | H |
| Ia1.080 | OCH₃ | CH₃ | 2-OCH₃-C₆H₄ | H |
| Ia1.081 | OCH₃ | CH₃ | 3-CF₃-C₆H₄ | H |
| Ia1.082 | OCH₃ | CH₃ | 4-OCF₃-C₆H₄ | H |
| Ia1.083 | OCH₃ | CH₃ | 2-CH₃-C₆H₄ | H |
| Ia1.084 | OCH₃ | CH₃ | 3-CH₃-C₆H₄ | H |
| Ia1.085 | OCH₃ | CH₃ | 2-COCH₃-C₆H₄ | H |
| Ia1.086 | OCH₃ | CH₃ | 3-COOCH₃-C₆H₄ | H |
| Ia1.087 | OCH₃ | CH₃ | 4-CF₃-C₆H₄ | H |
| Ia1.088 | OCH₃ | CH₃ | 2-CF₃-C₆H₄ | H |
| Ia1.089 | OCH₃ | CH₃ | 3-OCH₃-C₆H₄ | H |
| Ia1.090 | OCH₃ | CH₃ | 4-OCH₃-C₆H₄ | H |
| Ia1.091 | CF₃ | H | H | H |
| Ia1.092 | CF₃ | H | CH₃ | H |
| Ia1.093 | CF₃ | H | C₂H₅ | H |
| Ia1.094 | CF₃ | H | n-C₃H₇ | H |
| Ia1.095 | CF₃ | H | n-C₄H₉ | H |
| Ia1.096 | CF₃ | H | CHO | H |
| Ia1.097 | CF₃ | H | COCH₃ | H |
| Ia1.098 | CF₃ | H | COOCH₃ | H |
| Ia1.099 | CF₃ | H | OCH₃ | H |
| Ia1.100 | CF₃ | H | C₆H₅ | H |
| Ia1.101 | CF₃ | H | CH=NOCH₃ | H |
| Ia1.102 | CF₃ | H | C(CH₃)=NOCH₃ | H |
| Ia1.103 | CF₃ | H | 2-Furyl | CH₃ |
| Ia1.104 | CF₃ | H | 3-Furyl | CH₃ |
| Ia1.105 | CF₃ | H | 2-Thienyl | CH₃ |
| Ia1.106 | CF₃ | H | 3-Thienyl | CH₃ |
| Ia1.107 | CF₃ | H | 2-Pyridyl | CH₃ |
| Ia1.108 | CF₃ | H | 3-Pyridyl | CH₃ |
| Ia1.109 | CF₃ | H | 4-Pyridyl | CH₃ |
| Ia1.110 | CF₃ | H | 2-Thiazolyl | CH₃ |
| Ia1.111 | CF₃ | H | 4-Thiazolyl | CH₃ |
| Ia1.112 | CF₃ | H | 5-Thiazolyl | CH₃ |
| Ia1.113 | CF₃ | H | 2-Pyrrolyl | CH₃ |
| Ia1.114 | CF₃ | H | 3-Pyrrolyl | CH₃ |
| Ia1.115 | CF₃ | H | 4-Pyrrolyl | CH₃ |
| Ia1.116 | CF₃ | H | 3-Isoxazolyl | CH₃ |
| Ia1.117 | CF₃ | H | 4-Isoxazolyl | CH₃ |
| Ia1.118 | CF₃ | H | 5-Isoxazolyl | CH₃ |
| Ia1.119 | CF₃ | H | 2-Oxazolyl | CH₃ |
| Ia1.120 | CF₃ | H | 4-Oxazolyl | CH₃ |
| Ia1.121 | SO₂CH₃ | H | H | H |
| Ia1.122 | SO₂CH₃ | H | CH₃ | H |
| Ia1.123 | SO₂CH₃ | H | C₂H₅ | H |
| Ia1.124 | SO₂CH₃ | H | n-C₃H₇ | H |
| Ia1.125 | SO₂CH₃ | H | n-C₄H₉ | H |
| Ia1.126 | SO₂CH₃ | H | CHO | H |
| Ia1.127 | SO₂CH₃ | H | COCH₃ | H |
| Ia1.128 | SO₂CH₃ | H | COOCH₃ | H |
| Ia1.129 | SO₂CH₃ | H | OCH₃ | H |
| Ia1.130 | SO₂CH₃ | H | C₆H₅ | H |
| Ia1.131 | SO₂CH₃ | H | CH=NOCH₃ | H |
| Ia1.132 | SO₂CH₃ | H | C(CH₃)=NOCH₃ | H |
| Ia1.133 | SO₂CH₃ | C₂H₅ | 5-Oxazolyl | H |
| Ia1.134 | SO₂CH₃ | C₂H₅ | 3-Pyrazolyl | H |
| Ia1.135 | SO₂CH₃ | C₂H₅ | 4-Pyrazolyl | H |
| Ia1.136 | SO₂CH₃ | C₂H₅ | 5-Pyrazolyl | H |
| Ia1.137 | SO₂CH₃ | C₂H₅ | 2-Imidazolyl | H |
| Ia1.138 | SO₂CH₃ | C₂H₅ | 4-Imidazolyl | H |
| Ia1.139 | SO₂CH₃ | C₂H₅ | 5-Imidazolyl | H |
| Ia1.140 | SO₂CH₃ | C₂H₅ | 2-Pyrimidinyl | H |
| Ia1.141 | SO₂CH₃ | C₂H₅ | 4-Pyrimidinyl | H |
| Ia1.142 | SO₂CH₃ | C₂H₅ | 5-Pyrimidinyl | H |
| Ia1.143 | SO₂CH₃ | C₂H₅ | 1,3-Dioxolan-2-yl | H |
| Ia1.144 | SO₂CH₃ | C₂H₅ | 1,3-Dioxolan-4-yl | H |
| Ia1.145 | SO₂CH₃ | C₂H₅ | 1,3-Dioxan-2-yl | H |
| Ia1.146 | SO₂CH₃ | C₂H₅ | 3-Pyridazinyl | H |
| Ia1.147 | SO₂CH₃ | C₂H₅ | 4-Pyridazinyl | H |
| Ia1.148 | SO₂CH₃ | C₂H₅ | 2-Pyrazinyl | H |
| Ia1.149 | SO₂CH₃ | C₂H₅ | 2-Pyridyl | H |
| Ia1.150 | SO₂CH₃ | C₂H₅ | 2-N-Methylpyrrolyl | H |
| Ia1.151 | NO₂ | H | H | H |
| Ia1.152 | NO₂ | H | CH₃ | H |
| Ia1.153 | NO₂ | H | C₂H₅ | H |
| Ia1.154 | NO₂ | H | n-C₃H₇ | H |
| Ia1.155 | NO₂ | H | n-C₄H₉ | H |
| Ia1.156 | NO₂ | H | CHO | H |
| Ia1.157 | NO₂ | H | COCH₃ | H |
| Ia1.158 | NO₂ | H | COOCH₃ | H |
| Ia1.159 | NO₂ | H | OCH₃ | H |
| Ia1.160 | NO₂ | H | C₆H₅ | H |
| Ia1.161 | NO₂ | H | CH=NOCH₃ | H |
| Ia1.162 | NO₂ | H | C(CH₃)=NOCH₃ | H |
| Ia1.163 | NO₂ | H | COOH | C₂H₅ |
| Ia1.164 | NO₂ | H | COOMe | C₂H₅ |
| Ia1.165 | NO₂ | H | COOC₂H₅ | C₂H₅ |
| Ia1.166 | NO₂ | H | COOCH₂C₆H₅ | C₂H₅ |
| Ia1.167 | NO₂ | H | COOC(CH₃)₃ | C₂H₅ |
| Ia1.168 | NO₂ | H | CH=NOCH₃ | C₂H₅ |
| Ia1.169 | NO₂ | H | CH=NOC₂H₅ | C₂H₅ |
| Ia1.170 | NO₂ | H | CH=NOCH₂C₆H₅ | C₂H₅ |
| Ia1.171 | NO₂ | H | CH=NOCH(CH₃)₂ | C₂H₅ |
| Ia1.172 | NO₂ | H | C(CH₃)=NOCH₃ | C₂H₅ |
| Ia1.173 | NO₂ | H | C(CH₃)=NOC₂H₅ | C₂H₅ |
| Ia1.174 | NO₂ | H | C(CH₃)=NOCH(CH₃)₂ | C₂H₅ |
| Ia1.175 | NO₂ | H | C(CH₃)=NOCH₂C₆H₅ | C₂H₅ |
| Ia1.176 | NO₂ | H | CH=NOCH₂-CH=CH₂ | C₂H₅ |
| Ia1.177 | NO₂ | H | CH=NOCH₂-C≡CH | C₂H₅ |
| Ia1.178 | NO₂ | H | CH₂-CHO | C₂H₅ |
| Ia1.179 | NO₂ | H | CH₂-CH=NOCH₃ | C₂H₅ |
| Ia1.180 | NO₂ | H | CH₂-CH=NOC₂H₅ | C₂H₅ |

- Ebenso insbesondere außerordentlich bevorzugt sind die Verbindungen Ia2, insbesondere die Verbindungen Ia2.001-Ia2.180, die sich von den entsprechenden Verbindungen Ia1.001-Ia1.180 dadurch unterscheiden, daß R¹⁶ für Methyl steht: 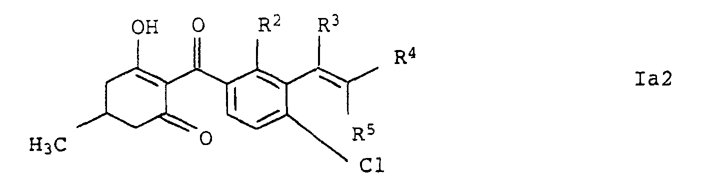
- Ebenso insbesondere außerordentlich bevorzugt sind die Verbindungen Ia3, insbesondere die Verbindungen Ia3.001-Ia3.180, die sich von den entsprechenden Verbindungen Ia1.001-Ia1.180 dadurch unterscheiden, daß R¹⁶ und R¹⁷ jeweils für Methyl stehen: 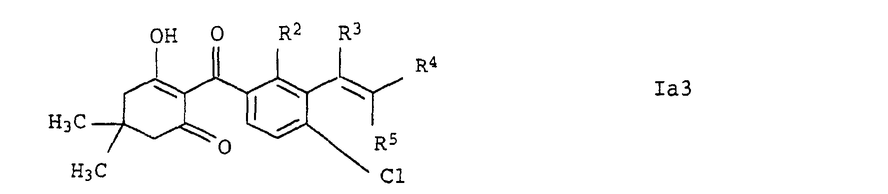
- Ebenso insbesondere außerordentlich bevorzugt sind die Verbindungen Ia4, insbesondere die Verbindungen Ia4.001-Ia4.180, die sich von den entsprechenden Verbindungen Ia1.001-Ia1.180 dadurch unterscheiden, daß R¹⁸ und R¹⁹ jeweils für Methyl stehen: 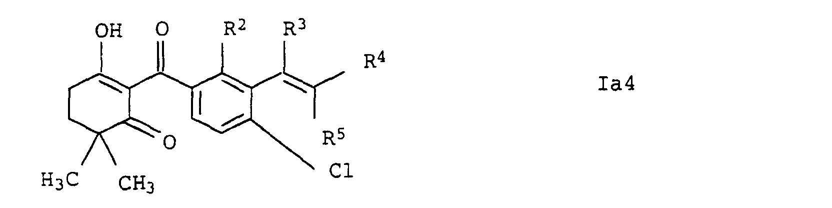
- Ebenso insbesondere außerordentlich bevorzugt sind die Verbindungen Ia5, insbesondere die Verbindungen Ia5.001-Ia5.180, die sich von den entsprechenden Verbindungen Ia1.001-Ia1.180 dadurch unterscheiden, daß die CR¹⁶R¹⁷-Einheit durch C=O ersetzt ist: 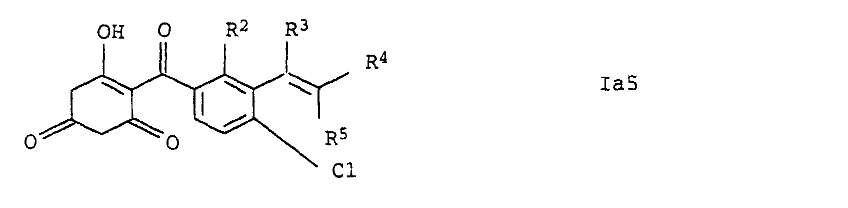
- Ebenso insbesondere außerordentlich bevorzugt sind die Verbindungen Ia6, insbesondere die Verbindungen Ia6.001-Ia6.180, die sich von den entsprechenden Verbindungen Ia1.001-Ia1.180 dadurch unterscheiden, daß R¹⁴, R¹⁸ und R¹⁹ jeweils für Methyl stehen und die CR¹⁶R¹⁷-Einheit durch C=O ersetzt ist: 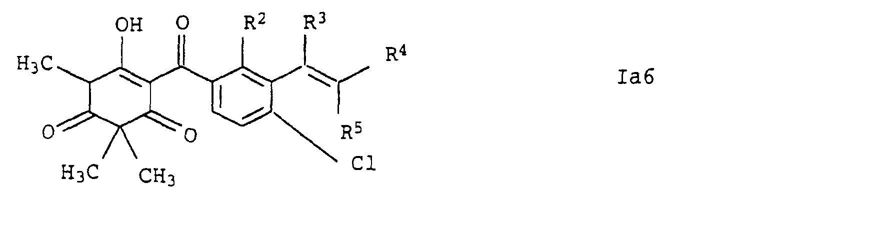
- Ebenso insbesondere außerordentlich bevorzugt sind die Verbindungen Ia7, insbesondere die Verbindungen Ia7.001-Ia7.180, die sich von den entsprechenden Verbindungen Ia1.001-Ia1.180 dadurch unterscheiden, daß R¹⁴, R¹⁵, R¹⁸ und R¹⁹ jeweils für Methyl stehen und die CR¹⁶R¹⁷-Einheit durch C=O ersetzt ist: 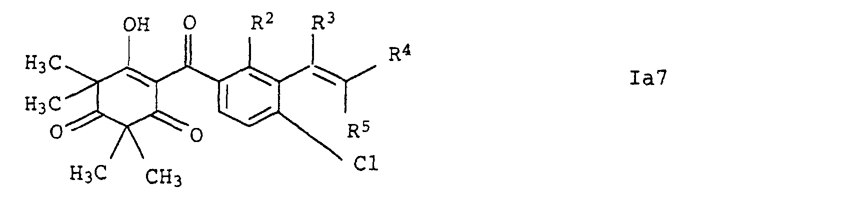
- Ebenso insbesondere außerordentlich bevorzugt sind die Verbindungen Ia8, insbesondere die Verbindungen Ia8.001-Ia8.180, die sich von den entsprechenden Verbindungen Ia1.001-Ia1.180 dadurch unterscheiden, daß R¹ für Nitro steht: 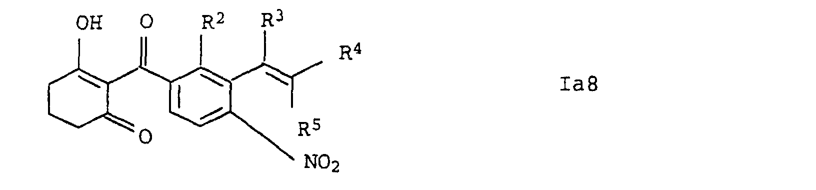
- Ebenso insbesondere außerordentlich bevorzugt sind die Verbindungen Ia9, insbesondere die Verbindungen Ia9.001-Ia9.180, die sich von den entsprechenden Verbindungen Ia1.001-Ia1.180 dadurch unterscheiden, daß R¹ für Nitro und R¹⁶ für Methyl stehen: 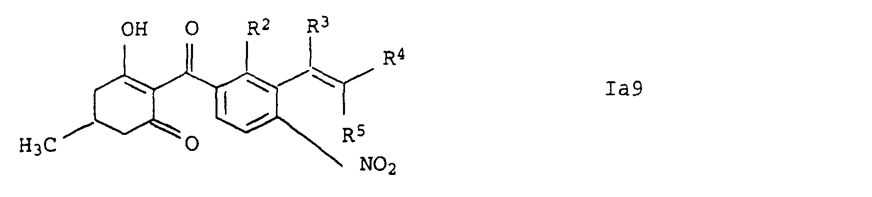
- Ebenso insbesondere außerordentlich bevorzugt sind die Verbindungen Ia10, insbesondere die Verbindungen Ia10.001-Ia10.180, die sich von den entsprechenden Verbindungen Ia1.001-Ia1.180 dadurch unterscheiden, daß R¹ für Nitro und R¹⁶ und R¹⁷ jeweils für Methyl stehen: 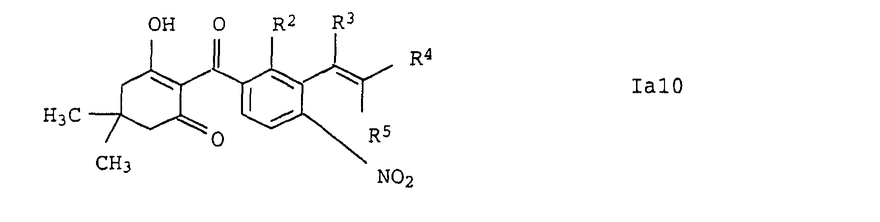
- Ebenso insbesondere außerordentlich bevorzugt sind die Verbindungen Ia11, insbesondere die Verbindungen Ia11.001-Ia11.180, die sich von den entsprechenden Verbindungen Ia1.001-Ia1.180 dadurch unterscheiden, daß R¹ für Nitro und R¹⁸ und R¹⁹ jeweils für Methyl stehen: 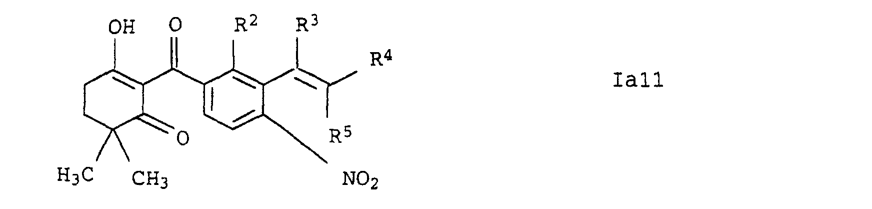
- Ebenso insbesondere außerordentlich bevorzugt sind die Verbindungen Ia12, insbesondere die Verbindungen Ia12.001-Ia12.180, die sich von den entsprechenden Verbindungen Ia1.001-Ia1.180 dadurch unterscheiden, daß R¹ für Nitro steht und die CR¹⁶R¹⁷-Einheit durch C=O ersetzt ist: 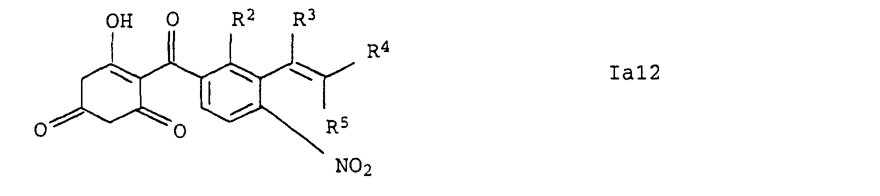
- Ebenso insbesondere außerordentlich bevorzugt sind die Verbindungen Ia13, insbesondere die Verbindungen Ia13.001-Ia13.180, die sich von den entsprechenden Verbindungen Ia1.001-Ia1.180 dadurch unterscheiden, daß R¹ für Nitro, R¹⁴, R¹⁸ und R¹⁹ jeweils für Methyl stehen und die CR¹⁶R¹⁷-Einheit durch C=O ersetzt ist: 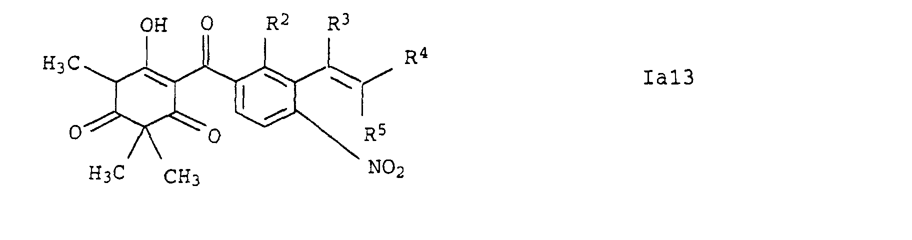
- Ebenso insbesondere außerordentlich bevorzugt sind die Verbindungen Ia14, insbesondere die Verbindungen Ia14.001-Ia14.180, die sich von den entsprechenden Verbindungen Ia1.001-Ia1.180 dadurch unterscheiden, daß R¹ für Nitro, R¹⁴, R¹⁵, R¹⁸ und R¹⁹ jeweils für Methyl stehen und die CR¹⁶R¹⁷-Einheit durch C=O ersetzt ist: 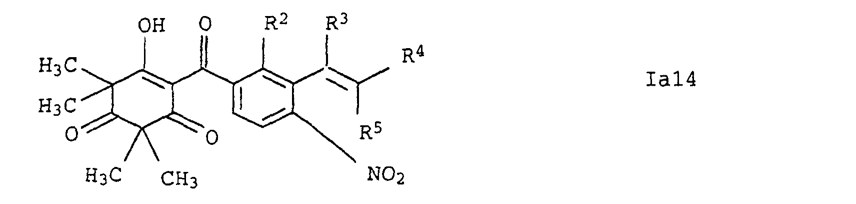
- Ebenso insbesondere außerordentlich bevorzugt sind die Verbindungen Ia15, insbesondere die Verbindungen Ia15.001-Ia15.180, die sich von den entsprechenden Verbindungen Ia1.001-Ia1.180 dadurch unterscheiden, daß R¹ für Methylsulfonyl steht: 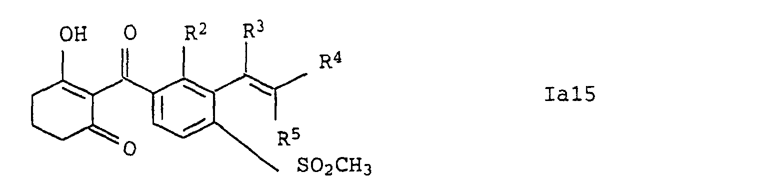
- Ebenso insbesondere außerordentlich bevorzugt sind die Verbindungen Ia16, insbesondere die Verbindungen Ia16.001-Ia16.180, die sich von den entsprechenden Verbindungen Ia1.001-Ia1.180 dadurch unterscheiden, daß R¹ für Methylsulfonyl und R¹⁶ für Methyl stehen: 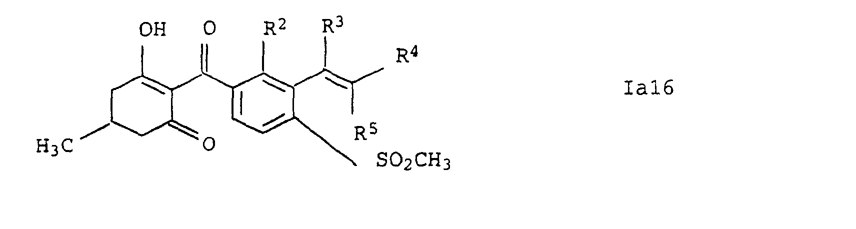
- Ebenso insbesondere außerordentlich bevorzugt sind die Verbindungen Ia17, insbesondere die Verbindungen Ia17.001-Ia17.180, die sich von den entsprechenden Verbindungen Ia1.001-Ia1.180 dadurch unterscheiden, daß R¹ für Methylsulfonyl und R¹⁶ und R¹⁷ jeweils für Methyl stehen: 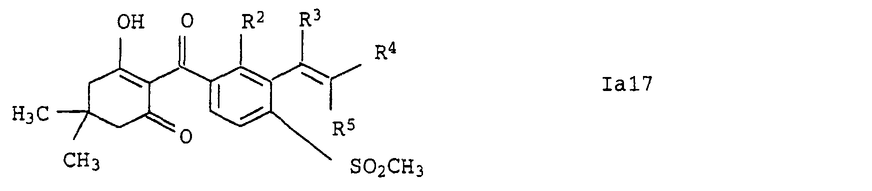
- Ebenso insbesondere außerordentlich bevorzugt sind die Verbindungen Ia18, insbesondere die Verbindungen Ia18.001-Ia18.180, die sich von den entsprechenden Verbindungen Ia1.001-Ia1.180 dadurch unterscheiden, daß R¹ für Methylsulfonyl und R¹⁸ und R¹⁹ jeweils für Methyl stehen: 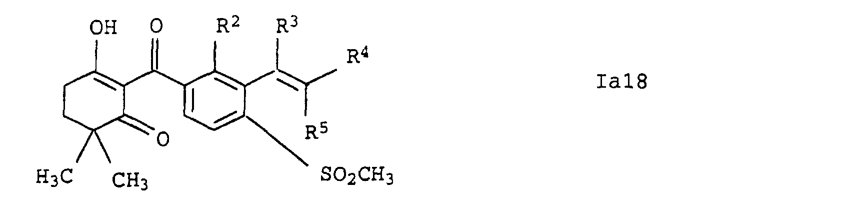
- Ebenso insbesondere außerordentlich bevorzugt sind die Verbindungen Ia19, insbesondere die Verbindungen Ia19.001-Ia19.180, die sich von den entsprechenden Verbindungen Ia1.001-Ia1.180 dadurch unterscheiden, daß R¹ für Methylsulfonyl steht und die CR¹⁶R¹⁷-Einheit durch C=O ersetzt ist: 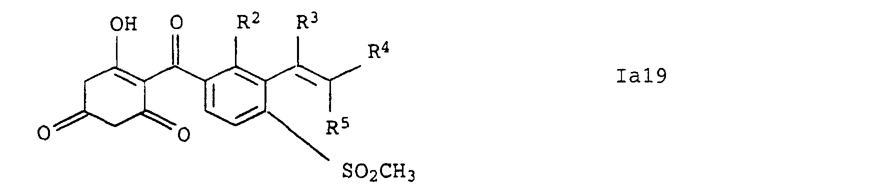
- Ebenso insbesondere außerordentlich bevorzugt sind die Verbindungen Ia20, insbesondere die Verbindungen Ia20.001-Ia20.180, die sich von den entsprechenden Verbindungen Ia1.001-Ia1.180 dadurch unterscheiden, daß R¹ für Methylsulfonyl, R¹⁴, R¹⁸ und R¹⁹ jeweils für Methyl stehen und die CR¹⁶R¹⁷-Einheit durch C=O ersetzt ist: 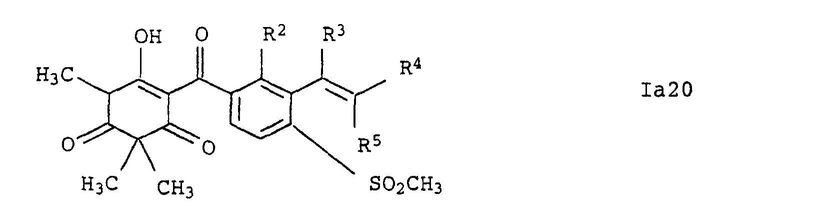
- Ebenso insbesondere außerordentlich bevorzugt sind die Verbindungen Ia21, insbesondere die Verbindungen Ia21.001-Ia21.180, die sich von den entsprechenden Verbindungen Ia1.001-Ia1.180 dadurch unterscheiden, daß R¹ für Methylsulfonyl, R¹⁴, R¹⁵, R¹⁸ und R¹⁹ jeweils für Methyl stehen und die CR¹⁶R¹⁷-Einheit durch C=O ersetzt ist: 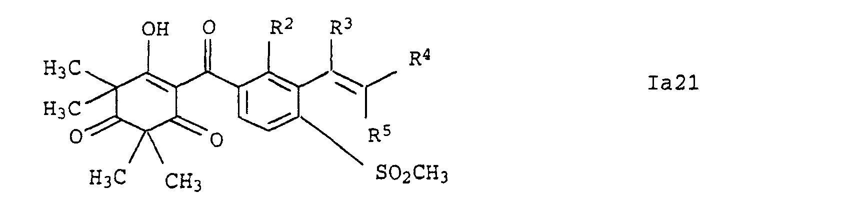
- Ebenso insbesondere außerordentlich bevorzugt sind die Verbindungen Ia22, insbesondere die Verbindungen Ia22.001-Ia22.180, die sich von den entsprechenden Verbindungen Ia1.001-Ia1.180 dadurch unterscheiden, daß R¹ für Trifluormethyl steht: 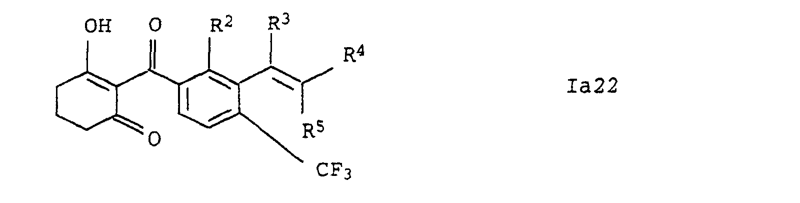
- Ebenso insbesondere außerordentlich bevorzugt sind die Verbindungen Ia23, insbesondere die Verbindungen Ia23.001-Ia23.180, die sich von den entsprechenden Verbindungen Ia1.001-Ia1.180 dadurch unterscheiden, daß R¹ für Trifluormethyl und R¹⁶ für Methyl stehen: 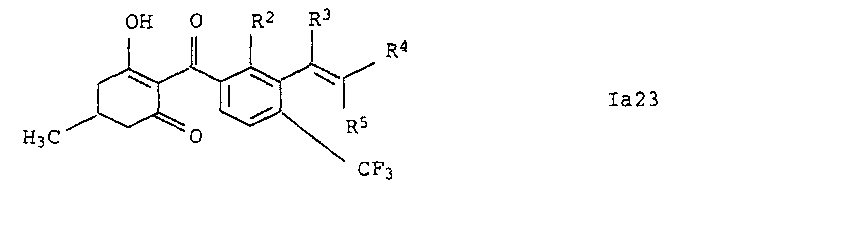
- Ebenso insbesondere außerordentlich bevorzugt sind die Verbindungen Ia24, insbesondere die Verbindungen Ia24.001-Ia24.180, die sich von den entsprechenden Verbindungen Ia1.001-Ia1.180 dadurch unterscheiden, daß R¹ für Trifluormethyl und R¹⁶ und R¹⁷ jeweils für Methyl stehen: 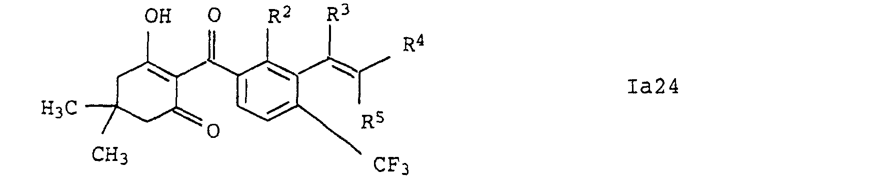
- Ebenso insbesondere außerordentlich bevorzugt sind die Verbindungen Ia25, insbesondere die Verbindungen Ia25.001-Ia25.180, die sich von den entsprechenden Verbindungen Ia1.001-Ia1.180 dadurch unterscheiden, daß R¹ für Trifluormethyl und R¹⁸ und R¹⁹ jeweils für Methyl stehen: 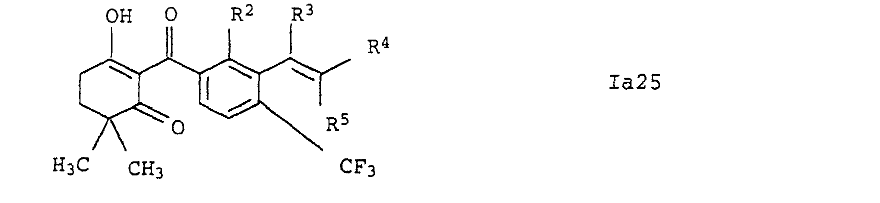
- Ebenso insbesondere außerordentlich bevorzugt sind die Verbindungen Ia26, insbesondere die Verbindungen Ia26.001-Ia26.180, die sich von den entsprechenden Verbindungen Ia1.001-Ia1.180 dadurch unterscheiden, daß R¹ für Trifluormethyl steht und die CR¹⁶R¹⁷-Einheit durch C=O ersetzt ist: 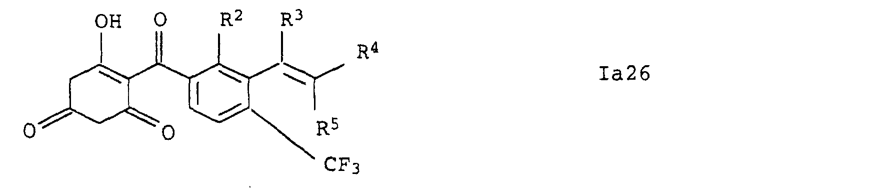
- Ebenso insbesondere außerordentlich bevorzugt sind die Verbindungen Ia27, insbesondere die Verbindungen Ia27.001-Ia27.180, die sich von den entsprechenden Verbindungen Ia1.001-Ia1.180 dadurch unterscheiden, daß R¹ für Trifluormethyl, R¹⁴, R¹⁸ und R¹⁹ jeweils für Methyl stehen und die CR¹⁶R¹⁷-Einheit durch C=O ersetzt ist: 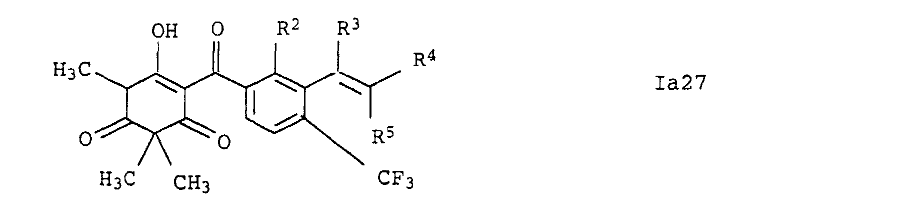
- Ebenso insbesondere außerordentlich bevorzugt sind die Verbindungen Ia28, insbesondere die Verbindungen Ia28.001-Ia28.180, die sich von den entsprechenden Verbindungen Ia1.001-Ia1.180 dadurch unterscheiden, daß R¹ für Trifluormethyl, R¹⁴, R¹⁵, R¹⁸ und R¹⁹ jeweils für Methyl stehen und die CR¹⁶R¹⁷-Einheit durch C=O ersetzt ist: 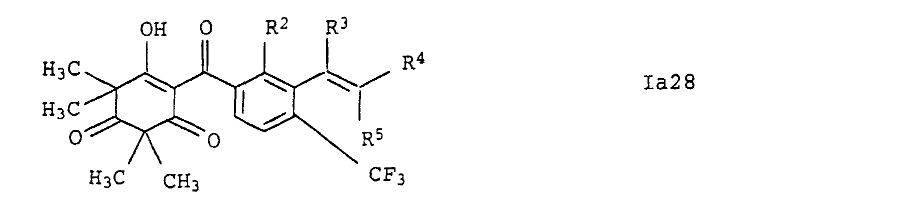
- Ebenso insbesondere außerordentlich bevorzugt sind die Verbindungen I, wobei
   - R¹: Halogen wie Chlor oder Brom, C₁-C₆-Alkylsulfonyl wie Methylsulfonyl oder Ethylsulfonyl; besonders bevorzugt chlor oder Methylsulfonyl;
   - R²: Halogen wie Chlor oder Brom; besonders bevorzugt Chlor;
   - R³: Wasserstoff;
   - R⁴: Wasserstoff, Cyano, C₁-C₆-Alkyl wie Methyl, Ethyl, Propyl, 2-Methylethyl, Butyl oder Pentyl, C₁-C₆-Alkoxy wie Methoxy, Ethoxy, C₁-C₆-Alkylthio wie Methylthio oder Ethylthio, Formyl, C₁-C₆-Alkylcarbonyl wie Methylcarbonyl oder Ethylcarbonyl, C₁-C₆-Alkoxycarbonyl wie Methoxycarbonyl oder Ethoxycarbonyl, -C(R¹²)=NR¹³;
   Heterocyclyl, Heterocycl-C₁-C₄-alkyl, Phenyl, Hetaryl, wobei die vier letztgenannten Reste ihrerseits substituiert sein können durch ein bis drei Halogenatome und/oder zwei bis drei Reste aus folgender Gruppe tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl, C₁-C₄-Alkoxycarbonyl, insbesondere C₁-C₄-Alkyl; besonders bevorzugt Wasserstoff, Cyano, Methyl, 2-Methylethyl, Pentyl, Methoxy, Methylthio, Formyl, Methylcarbonyl, Ethoxycarbonyl, -C(R¹²)=NR¹³, 2-Methyl-1,3-dioxolan-4-yl, 2,2-Dimethyl-1,3-dioxolan-4-yl, 1,3-Dioxan-2-yl, 1,3-Dioxan-2-ylmethyl, Phenyl, 2-Furyl, 3-Furyl, 2-Thienyl, 3-Thienyl;
   - R⁵: Wasserstoff, Halogen wie Chlor oder Brom oder C₁-C₆-Alkoxy wie Methoxy oder Ethoxy; besonders bevorzugt Wasserstoff, chlor oder Ethoxy;
   - R¹²: Wasserstoff, C₁-C₆-Alkyl wie Methyl oder Ethyl oder C₁-C₆-Alkoxycarbonyl wie Methoxycarbonyl oder Ethoxycarbonyl;
   besonders bevorzugt Wasserstoff, Methyl oder Methoxycarbonyl;
   - R¹⁴, R¹⁵,: R¹⁶, R¹⁷, R¹⁸ und R¹⁹ Wasserstoff oder C₁-C₄-Alkyl wie Methyl oder Ethyl;
   besonders bevorzugt Wasserstoff oder Methyl;
bedeuten;
oder die CR¹⁶R¹⁷-Einheit durch C=O ersetzt sein kann.

Die 2-(3-Alkenyl-benzoyl)-cyclohexan-1,3-dione der Formel I sind auf verschiedene Art und Weise erhältlich, beispielsweise nach folgendem Verfahren:

Umsetzung von Cyclohexandionen der Formel II mit einer aktivierten Carbonsäure IIIα oder einer Carbonsäure IIIβ, die vorzugsweise in situ aktiviert wird, zu dem Acylierungsprodukt IV und anschließende Umlagerung.

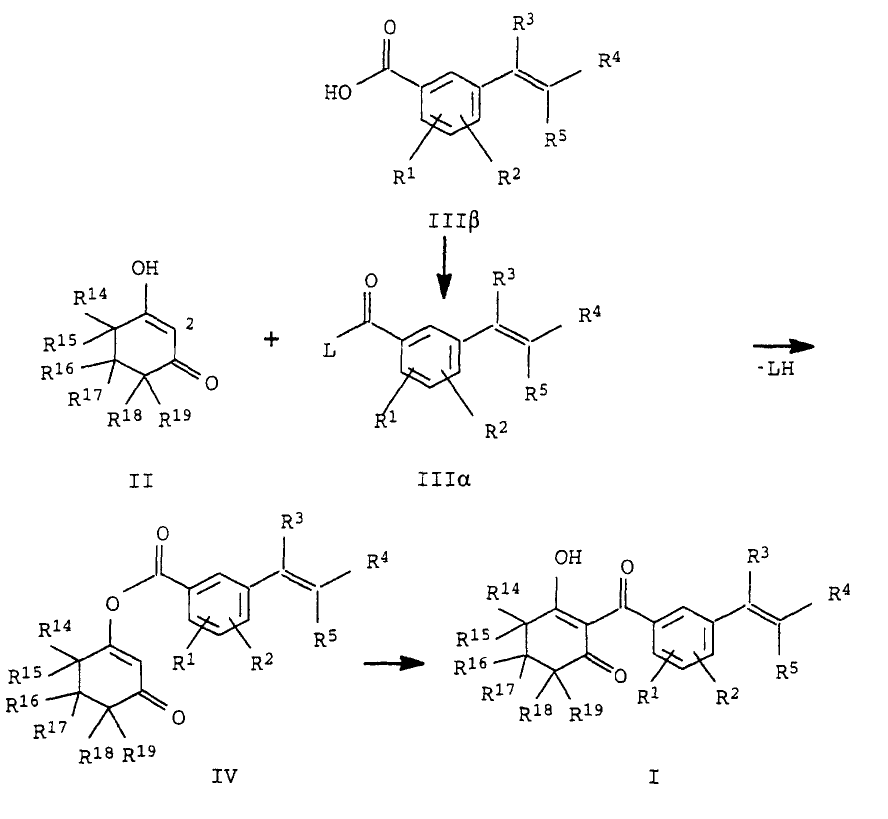

L steht für eine nucleophil verdrängbare Abgangsgruppe, wie Halogen, z.B. Brom, Chlor, Hetaryl, z.B. Imidazolyl, Pyridyl, Carboxylat, z.B. Acetat, Trifluoracetat etc.

Die aktivierte Carbonsäure kann direkt eingesetzt werden, wie im Fall der Carbonsäurehalogenide oder in situ erzeugt werden, z.B. mit Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel IV vorzugsweise durch Chromatographie gereinigt. Es ist aber auch möglich, den rohen Enolester der Formel IV ohne weitere Reinigung zur Umlagerung einzusetzen.

Die Umlagerung der Enolester der Formel IV zu den Verbindungen der Formel I erfolgt zweckmäßigerweise bei Temperaturen von 20 bis 40°C in einem Lösungsmittel und in Gegenwart einer Hilfsbase sowie gegebenenfalls mit Hilfe einer Cyanoverbindung als Katalysator.

Als Lösungsmittel können z.B. Acetonitril, Methylenchlorid, 1,2-Dichlorethan, Essigsäureethylester, Toluol oder Gemische hiervon verwendet werden. Bevorzugtes Lösungsmittel ist Acetonitril.

Geeignete Hilfsbasen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Enolester, eingesetzt werden. Bevorzugt wird Triethylamin verwendet, vorzugsweise in doppelt äquimolaren Verhältnis in Bezug auf den Enolester.

Als "Umlagerungskatalysator" kommen anorganische Cyanide, wie Natriumcyanid, Kaliumcyanid und organische Cyanoverbindungen, wie Acetoncyanhydrin, Trimethylsilylcyanid in Betracht. Sie werden üblicherweise in einer Menge von 1 bis 50 Molprozent, bezogen auf den Enolester, eingesetzt. Vorzugsweise werden Acetoncyanhydrin oder Trimethylsilylcyanid, z.B. in einer Menge von 5 bis 15, vorzugsweise 10 Molprozent, bezogen auf den Enolester, eingesetzt.

Die Aufarbeitung kann in an sich bekannter Weise erfolgen. Das Reaktionsgemisch wird z.B. mit verdünnter Mineralsäure, wie z.B. 5%ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Enolestern von Cyclohexan-1,3-dionen und für die cyanidkatalysierte Umlagerung der Enolester sind z.B. in EP-A 186 118, US 4 780 127 genannt).

Die als Ausgangsmaterialien verwendeten Cyclohexan-1,3-dione der Formel II sind bekannt oder können nach an sich bekannten Verfahren hergestellt werden (z.B. EP-A 71 707, EP-A 142 741, EP-A 243 313, US 4 249 937; WO 92/13821).

Die aktivierten Carbonsäuren IIIα, die nicht in-situ erzeugt werden können auf an sich bekannte Art und Weise dargestellt werden. Beispielsweise können Carbonsäurehalogenide der Formel IIIα (mit L = Halogen) in Analogie zu literaturbekannten Methoden (vgl. L.G. Fieser, M. Fieser "Reagents for Organic Synthesis", Bd. I, S. 767-769 (1967)) durch Umsetzung von Benzoesäuren der Formel IIIβ mit Halogenierungsreagentien wie Thionylchlorid, Thionylbromid, Phosgen, Diphosgen, Triphosgen, Oxalylchlorid, Oxalylbromid synthetisiert werden.

Die 3-Alkenyl-benzoesäuren der Formel IIIβ sind literaturbekannt oder können in Analogie zu an sich literaturbekannten Methoden u.a. durch Verseifung der entsprechenden 3-Alkenyl-benzoesäureester (mit M = C₁-C₆-Alkoxy) der Formel IIIγ erhalten werden.

Auch die 3-Alkenyl-benzoesäureester (mit M = C₁-C₆-Alkoxy) der Formel IIIγ sind literaturbekannt oder sind auf verschiedene Art und Weise erhältlich, beispielsweise nach folgenden Verfahren:

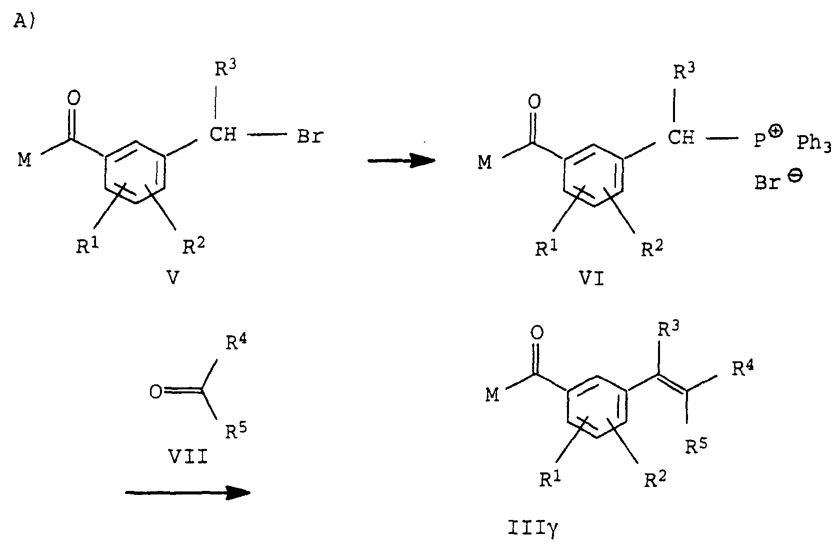

Durch Wittig-Reaktion von Phosphoniumsalzen der Formel VI mit Aldehyden oder Ketonen (VII) können auf an sich bekannte Art und Weise (J. March, "Advanced Organic Chemistry", 3. Auflage, S. 864 ff, Wiley-Interscience Publication, 1985) die Verbindungen der allgemeinen Formel IIIγ erhalten werden.

Die Phosphoniumsalze der Formel VI sind auf an sich bekannte Art und Weise (J. March, "Advanced Organic Chemistry", 3. Auflage, S. 377 ff, Wiley-Interscience Publication, 1985) aus den Bromverbindungen der Formel V zugänglich.

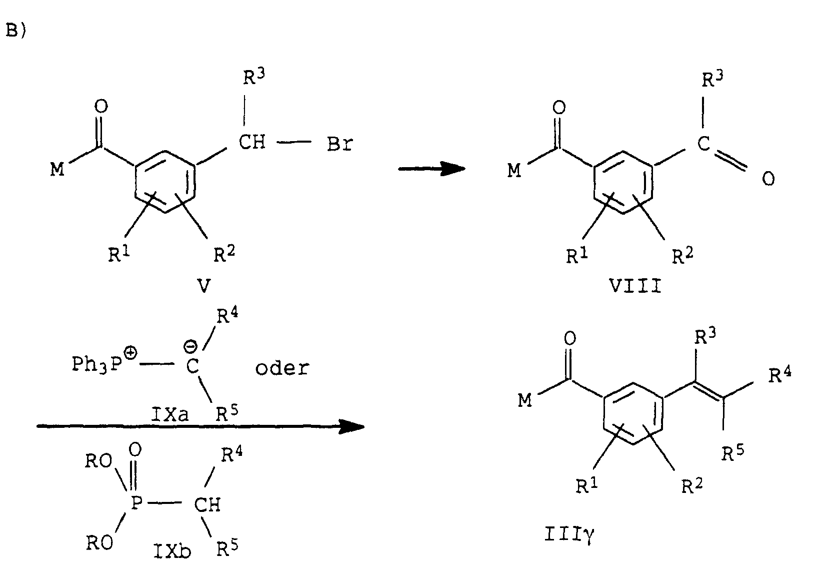

Die Verbindungen der allgemeinen Formel IIIγ können auch durch Wittig-Reaktion bzw. Horner-Emmons-Reaktion von Aldehyden oder Ketonen der Formel VIII mit Phosphoniumsalzen IXa (siehe auch A) bzw. Phosphonaten IXb (J. March, "Advanced Organic Chemistry", 3. Auflage, S. 867 ff., Wiley-Interscience Publication, 1985) erhalten werden.

Die Verbindungen der Formel VIII sind auf an sich bekannte Art und Weise (J. March, "Advanced Organic Chemistry", 3. Auflage, S. 1105 ff, Wiley-Interscience Publication, 1985) durch Oxidation von Bromverbindungen der Formel V zugänglich.

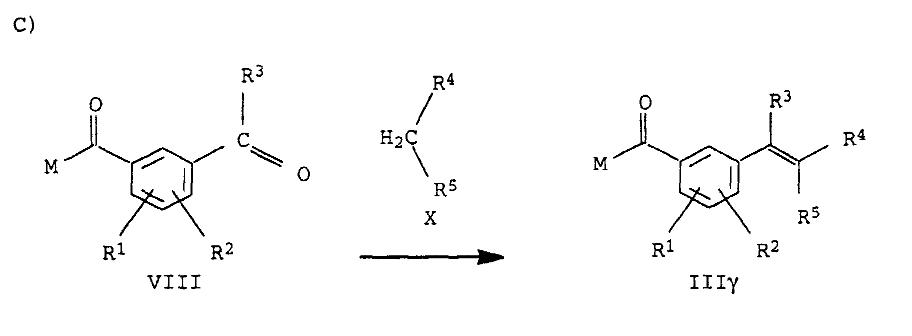

Durch Aldolkondensation und verwandten Reaktionen von Aldehyden oder Ketonen der Formel VIII können ebenfalls auf an sich bekannte Art und Weise (J. March, "Advanced Organic Chemistry", 3. Auflage, S. 849 ff, Wiley-Interscience Publications, 1985) die Verbindungen der allgemeinen Formel IIIγ erhalten werden.

### Herstellungsbeispiele

### 2-[2',4'-Dichlor-3'-(prop-1"-en-1"-yl)-benzoyl]-1,3-cyclohexandion (Verbindung 2.1)

Zu einer Lösung von 4,0 g (16 mmol) 2,4-Dichlor-3-(prop-1'-en-1'-yl)benzoylchlorid in 50 ml Acetonitril wurden 17 g (16 mmol) Cyclohexandion und 2,2 ml (16 mmol) Triethylamin gegeben. Nach 12 Stunden Rühren bei Raumtemperatur wurde in 500 ml Wasser gegossen und dreimal mit Essigsäureethylester extrahiert. Nach Trocknen über Natriumsulfat wurde das Lösungsmittel am Vakuum entfernt und der Rückstand mittels Chromatographie an Kieselgel (Cyclohexan/Essigsäureethylester = 9/1) gereinigt. Der so erhaltene Enolester wurde in 100 ml Acetonitril gelöst und mit 1,0 g (10 mmol) Trimethylsilylcyanid und 1,3 ml Triethylamin versetzt. Nach 12 Stunden Rühren bei Raumtemperatur wurde am Vakuum aufkonzentriert und der Rückstand in 200 ml 10 %ige Sodalösung gegossen. Danach wurde einmal mit Methyl-t-butylether extrahiert und die wäßrige Phase mit 10 %iger Salzsäure auf pH 3 gestellt. Nach Extraktion mit Essigsäureethylester und Trocknen über Natriumsulfat wurde das Lösungsmittel am Vakuum entfernt. Es verbleiben 0,6 g eines schwach gelben Pulvers vom Fp. 115 - 120°C. ¹H-NMR (CDCl₃/δ in ppm): 17,0 (1H) ; 7,45 (1H); 6,95 (1H); 6,30 (1H); 6,15 (1H); 2,75 (2H); 2,45 (2H); 2,05 (2H); 1,90 (3H).

### 2-[2',4'-Dichlor-3'-(2"-methoxyethen-1"-yl)benzoyl]-1,3-cyclohexandion (Verbindung 2.7)

2,5 g (10 mmol) 2,4-Dichlor-3-(2'-methoxyethen-1'-yl)benzoesäure wurden unter Stickstoff in 50 ml Acetonitril suspendiert und mit 11 g (10 mmol) Cyclohexandion und 2,1 g (10 mmol) Dicyclohexylcarbodiimid versetzt. Nach 12 Stunden Rühren bei Raumtemperatur wurde der ausgefallene Harnstoff abgesaugt und das Lösungsmittel am Vakuum entfernt. Der Rückstand wurde mittels Chromatographie an Kieselgel (Cyclohexan/Essigsäureethylester = 9/1) gereinigt. Der so erhaltene Enolester wurde in 50 ml Acetonitril gelöst und mit 0,4 g (4,4 mmol) Trimethylsilylcyanid und 0,4 g (4,4 mmol) Triethylamin versetzt. Nach 3 Stunden Rühren bei Raumtemperatur wurde auf ein Drittel des Volumens eingeengt und in 200 ml 5 %ige Sodalösung gegossen. Nun wurde einmal mit wenig Methyl-t-butylether extrahiert und mit 10 %iger Salzsäure auf pH 3 gestellt. Nach Extraktion mit Essigsäureethylester und Einengen am Vakuum verblieben 1,0 g eines gelben Harzes.
¹H-NMR (CDCl₃/δ in ppm): 17,0 (1H); 7,35 (1H); 7,10 (1H); 6,90 (1H); 5,80 (1H); 3,75 (3H); 2,80 (2H); 2,40 (2H); 2,05 (2H).

In der folgenden Tabelle 2 sind neben den voranstehend beschriebenen 2-(3-Alkenyl-benzoyl)-cyclohexan-1,3-dionen der Formel I noch weitere aufgeführt, die in analoger Weise hergestellt wurden:

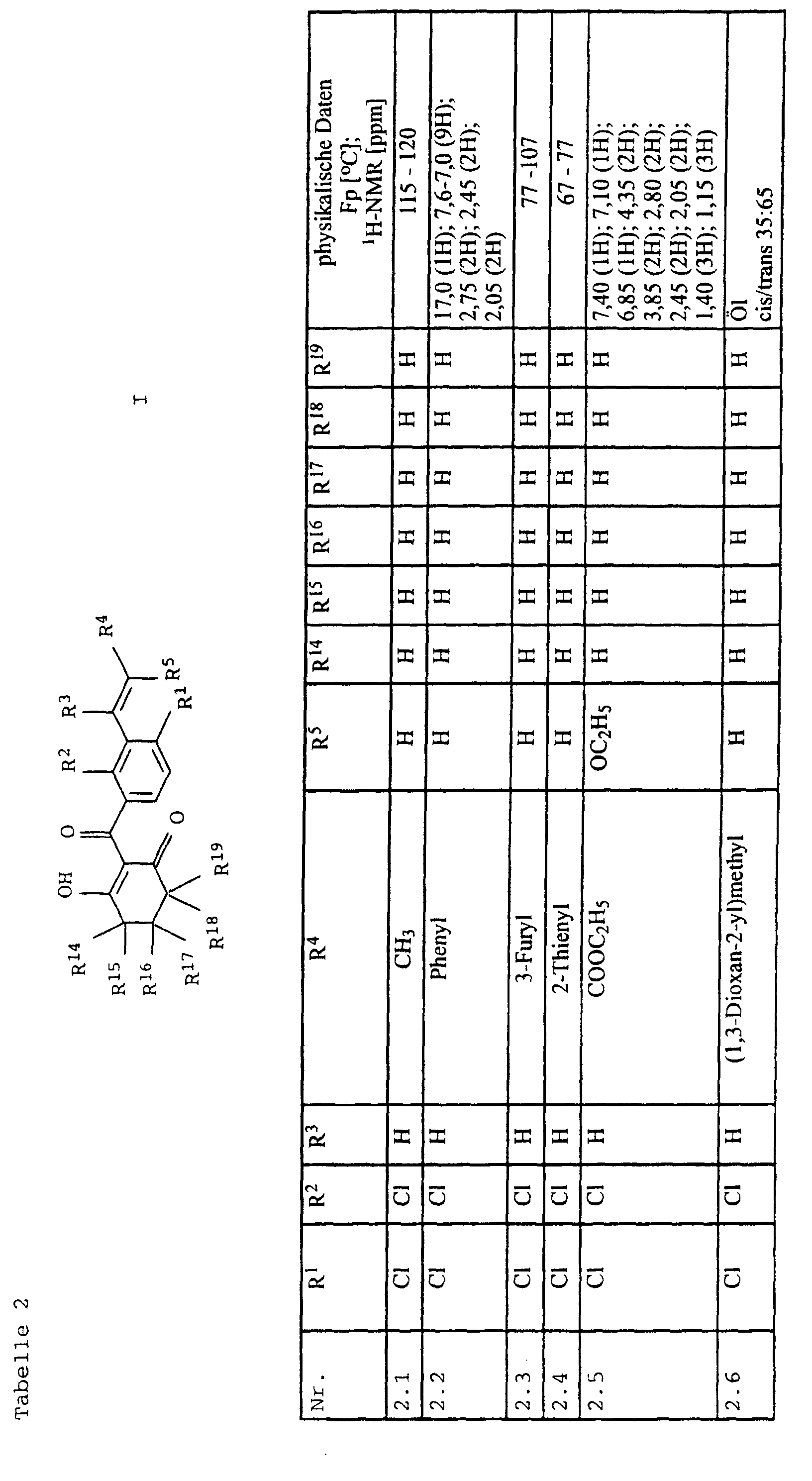

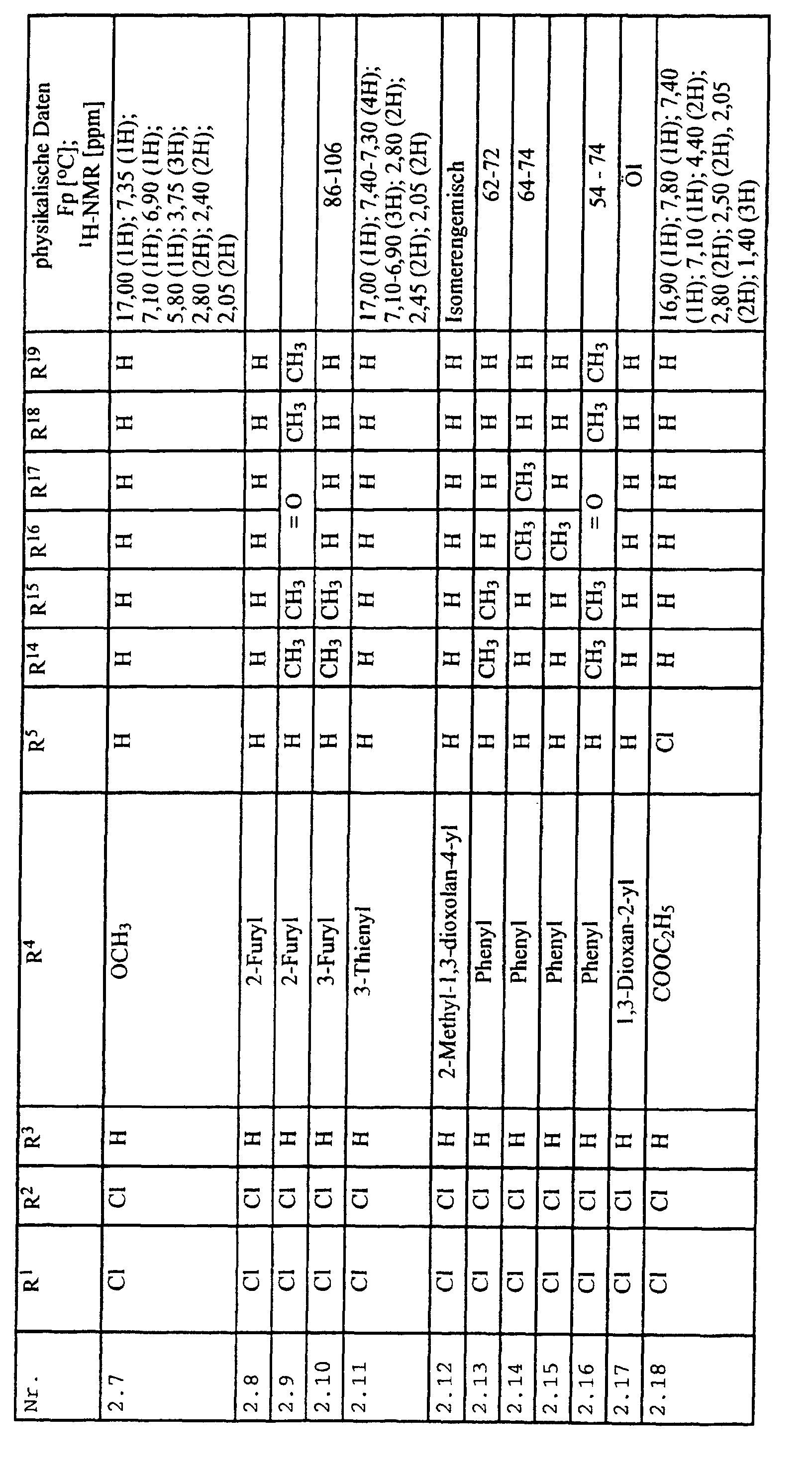

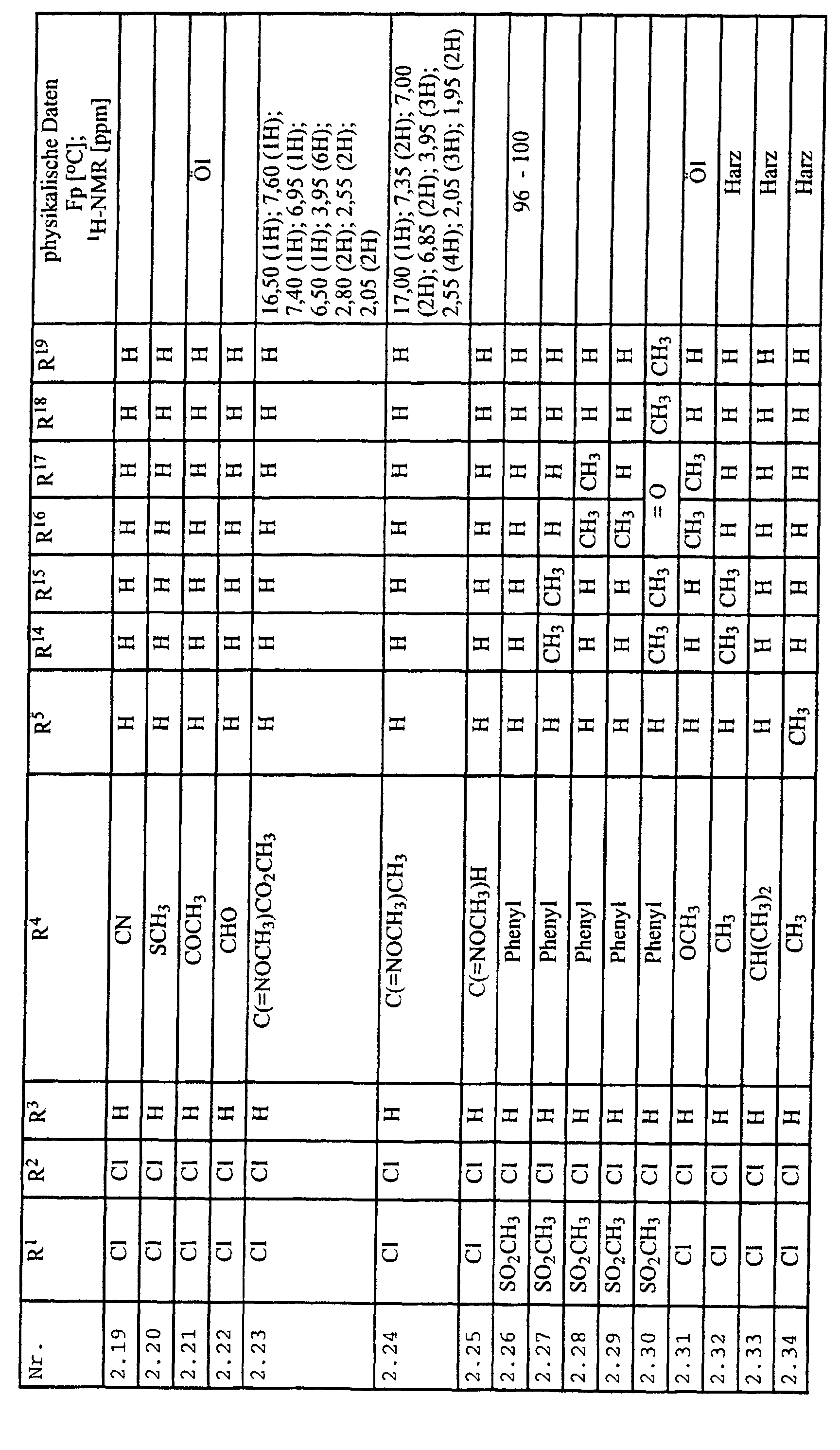

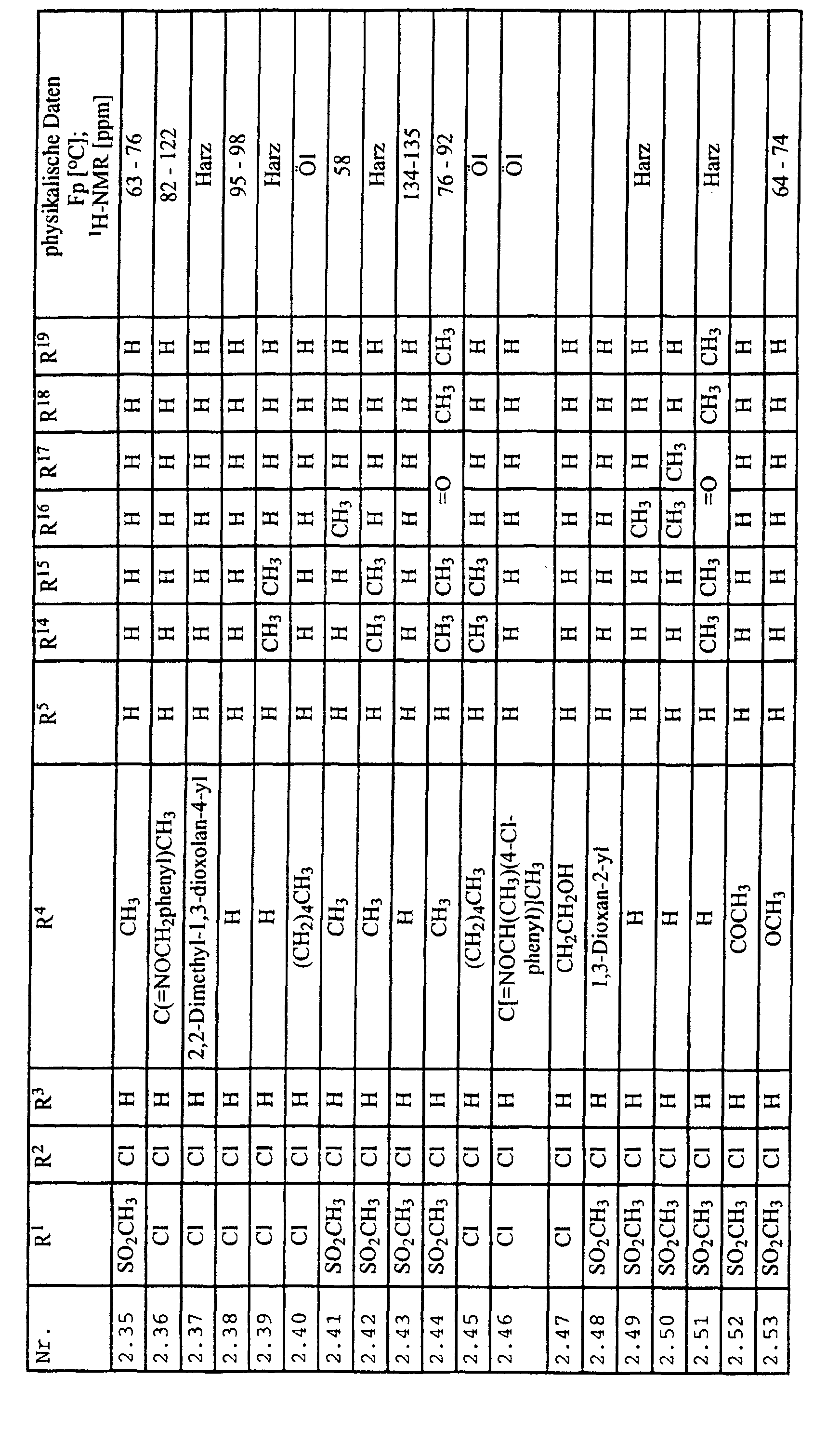

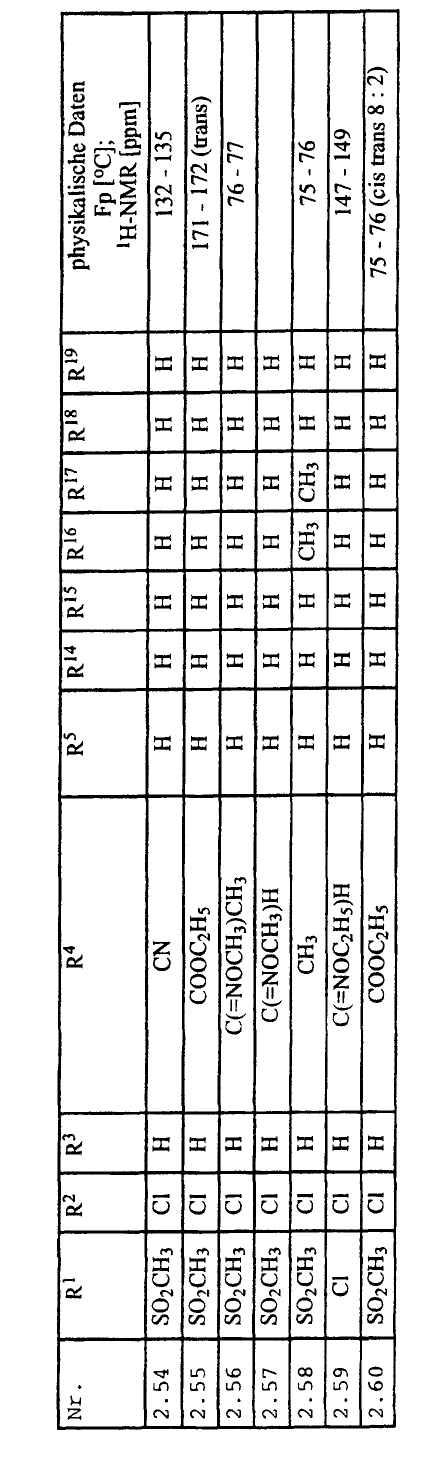

Nachfolgend sind die Synthesen einiger Ausgangsstoffe aufgeführt:

### 2-Chlor-4-methylsulfonyl-3-(2'-phenylethen-1'-yl)-benzoesäure (Verbindung 3.02)

### Stufe a) 2-Chlor-4-methylsulfonyl-3-(2'-phenylethen-1'-yl)-benzoesäuremethylester (Verbindung 3.01)

Zu einer Lösung von 52,0 g (120 mmol) Benzyltriphenylphosphoniumbromid in 400 ml Tetrahydrofuran wurden 10,1 g (90 mmol) Kalium-tert.-butylat gegeben. Nach 30 Minuten Rühren bei Raumtemperatur wurden 16,6 g (60 mmol) 2-Chlor-3-formyl-4-methylsulfonyl-benzoesäuremethylester in 100 ml Tetrahydrofuran zugetropft und weitere 3 Stunden bei Raumtemperatur gerührt. Danach wurde das Reaktionsgemisch in 500 ml Wasser eingerührt und mit Methyl-t-butylether extrahiert. Nach Entfernen des Lösungsmittels im Vakuum wurde der Rückstand mit Diethylether digeriert und das ausgefallene Triphenylphosphinoxid abgesaugt. Der Rückstand wurde mittels Chromatographie an Kieselgel (Cyclohexan/Essigsäureethylester = 95/5 bis 1/1) gereinigt. Man erhielt 10,9 g (52 % d.Th.) eines leicht gelben Öls, das langsam erstarrte.
¹H-NMR (CDCl₃/δ in ppm): 8,15 (1H); 7,70 (1H); 7,60-7,30 (7H); 7,00 (1H); 4,00 (3H); 3,10 (3H).

### Stufe b) 2-Chlor-4-methylsulfonyl-3-(2'-phenylethen-1'-yl)-benzoesäure

10,0 g (28 mmol) 2-Chlor-3-(2'-phenylethen-1'-yl)-4-methylsulfonyl-benzoesäuremethylester wurden in 200 ml Tetrahydrofuran/Methanol (1/1) gelöst und mit 35,2 g 10 %iger Natronlauge versetzt. Danach wurde 12 Stunden bei Raumtemperatur gerührt und anschließend das Lösungsmittel im Vakuum entfernt. Der Rückstand wurde mit 400 ml Wasser versetzt und mit Essigsäureethylester gewaschen. Nun wurde mit 10 %iger Salzsäure ein pH-Wert von 1 eingestellt und der gebildete Niederschlag abgesaugt. Nach Trocknen verblieben 9,4 g (97 % d.Th.) eines weißen Pulvers vom Fp.: 232-233°C.
¹H-NMR (CDCl₃/δ in ppm): 8,20 (1H); 7,90 (1H); 7,55 (2H); 7,40 (4H); 7,00 (1H); 3,10 (3H).

### 2,4-Dichlor-3-[2'-(2"-furyl)ethen-1'-yl]-benzoesäuremethylester (Verbindung 3.05)

### Stufe a) 2,4-Dichlor-3-methyl-acetophenon

Zu einer Lösung von 502,0 g (3,12 mol) 2,6-Dichlortoluol und 408,0 g (3,06 mol) Aluminiumtrichlorid wurden bei 100°C unter Rühren 235,0 g (3,0 mol) Acetylchlorid über einen Zeitraum von 2 Stunden zugetropft. Nach 2 Stunden Rühren bei 100-105°C kühlte man ab und goß das Reaktionsgemisch auf 3 l Eis und 1 l Wasser. Der dabei ausgefallene Feststoff wurde abgesaugt und mit Wasser neutral gewaschen. Nach dem Trocknen bei 40°C erhielt man 500,0 g 2,4-Dichlor-3-methyl-acetophenon als Rohprodukt, das anschließend im Hochvakuum destilliert wurde.
(Sdp.: 121-128°C (4 mbar))

### Stufe b) 2,4-Dichlor-3-methyl-benzoesäure

In eine Lösung von 520,0 g (13 mol) Natriumhydroxid in 2600 ml Wasser wurden bei 0-10°C zunächst 655,2 g (4,1 mol) Brom und anschließend 203,0 g (1,0 mol) 2,4-Dichlor-3-methyl-acetophenon in 1300 ml 1,4-Dioxan zugetropft. Nach 12 Stunden Rühren trennte man die organische Phase ab, versetzte die wäßrige Phase mit einer 30 %igen Lösung, dargestellt aus Natriumpyrosulfit und Wasser, und stellte mit Salzsäure einen pH-Wert von 1 ein. Der ausgefallene Niederschlag wurde abgesaugt, mit Wasser gewaschen und am Vakuum bei 60°C getrocknet. Man erhielt 197,0 g 2,4-Dichlor-3-methyl-benzoesäure.
(Fp.: 173-175°C)

### Stufe c) 2,4-Dichlor-3-methyl-benzoesäuremethylester

Zu einer Lösung von 424,0 g (2 mol) 2,4-Dichlor-3-methylbenzoesäure und 1500 ml Methanol wurden 60 ml konz. Schwefelsäure getropft. Nach 5 Stunden Erhitzen unter Rückfluß wurde das Reaktionsgemisch abgekühlt, im Vakuum eingeengt und anschließend in 1000 ml Methylenchlorid aufgenommen. Die organische Phase wurde mit Wasser, anschließend mit 5 %iger Natriumhydrogencarbonat-Lösung und dann wiederum mit Wasser gewaschen, getrocknet und am Vakuum eingeengt. Man erhielt 401,0 g 2,4-Dichlor-3-methyl-benzoesäuremethylester.
(Sdp: 103-107°C (1-1,5 mbar))

### Stufe d) 3-Brommethyl-2,4-dichlor-benzoesäuremethylester

Zu einer Lösung von 84,0 g (0,38 mol) 2,4-Dichlor-3-methyl-benzoesäuremethylester und 67,6 g (0,38 mol) N-Bromsuccinimid in 380 ml Tetrachlorkohlenstoff wurde 1,0 g Azobisisobutyronitril gegeben. Nach 3,5 Stunden Erhitzen unter Rückfluß wurde das Reaktionsgemisch abgekühlt und der gebildete Niederschlag abgesaugt. Das Filtrat wurde am Vakuum eingeengt und der resultierende Rückstand aus Methyl-t-butylether ausgerührt. Man erhielt 108,0 g 3-Brommethyl-2,4-dichlor-benzoesäuremethylester.
(Fp.: 51-54°C)

### Stufe e) (2,6-Dichlor-3-methoxycarbonyl)-benzyl-triphenylphosphoniumbromid

80,65 g (262 mmol) 3-Brommethyl-2,4-dichlor-benzoesäuremethylester wurden in 800 ml Toluol gelöst und mit 68,7 g (262 mmol) Triphenylphosphin versetzt. Nach 9 Stunden Rühren am Rückfluß kühlte man ab und saugte den gebildeten Niederschlag ab. Nach Trocknen verblieben 129,0 g (89 % d.Th.) eines hellbeigen Pulvers.
(Fp.: 238-239°C)

### Stufe f) 2,4-Dichlor-3-(2'-(2"-furyl)ethen-1'-yl)benzoesäuremethylester

28,0 g (50 mmol) (2,6-Dichlor-3-methoxycarbonyl)-benzyltriphenylphosphoniumbromid wurden in 200 ml Tetrahydrofuran suspendiert und bei 0°C mit 5,6 g (50 mmol) Kaliumt-butylat versetzt. Anschließend wurde auf -20°C abgekühlt und eine Lösung von 6,2 g (65 mmol) Furfurol in 50 ml Tetrahydrofuran zugetropft. Man erwärmte auf Raumtemperatur und rührte weitere 12 Stunden. Das Reaktionsgemisch wurde in 200 ml Wasser eingerührt und mit Methyl-t-butylether extrahiert. Nach Entfernen des Lösungsmittels im Vakuum wurde der Rückstand mit Diethylether digeriert und das ausgefallene Triphenylphosphinoxid abgesaugt. Der Rückstand wurde mittels Chromatographie an Kieselgel (Cyclohexan/Essigester = 98/2 bis 90/10) gereinigt. Man erhielt 8,2 g (55 % d.Th.) eines gelben Öls.
¹H-NMR (CDCl₃/δ in ppm): 7,55 (1H); 7,50 (1H); 7,40 (1H); 7,05 (1H); 6,95 (1H); 6,45 (2H); 3,95 (3H).

### 2,4-Dichlor-3-(3'-methoxycarbonyl-3'-methoxyimino-prop-1'-en-1'yl)benzoesäuremethylester (Verbindung 3.13)

Zu 17,9 g (75 mmol) (2-Methoxycarbonyl-2-methoxyimino-ethyl)dimethylphosphonat in 150 ml Tetrahydrofuran wurden 1,9 g (75 mmol) Natriumhydrid gegeben und 2 Stunden bei Raumtemperatur gerührt. Anschließend wurden 11,7 g (50 mmol) 2,4-Dichlor-3-formylbenzoesäuremethylester in 50 ml Tetrahydrofuran zugetropft und weitere 12 Stunden bei Raumtemperatur gerührt. Nach Aufnehmen des Reaktionsansatzes in Wasser wurde mit Methyl-t-butylether extrahiert, getrocknet und das Lösungsmittel im Vakuum entfernt. Der Rückstand wurde mit Diethylether digeriert und der Niederschlag abgetrennt. Nach Trocknen verblieben 11,3 g (65 % d.Th.) eines weißen Pulvers.
(Fp.: 96 - 97°C)

In nachfolgender Tabelle 3 sind neben den voranstehend beschriebenen Verbindungen weitere Benzoesäurederivate der Formel IIIa aufgeführt, die in analoger Weise hergestellt wurden oder herstellbar sind.

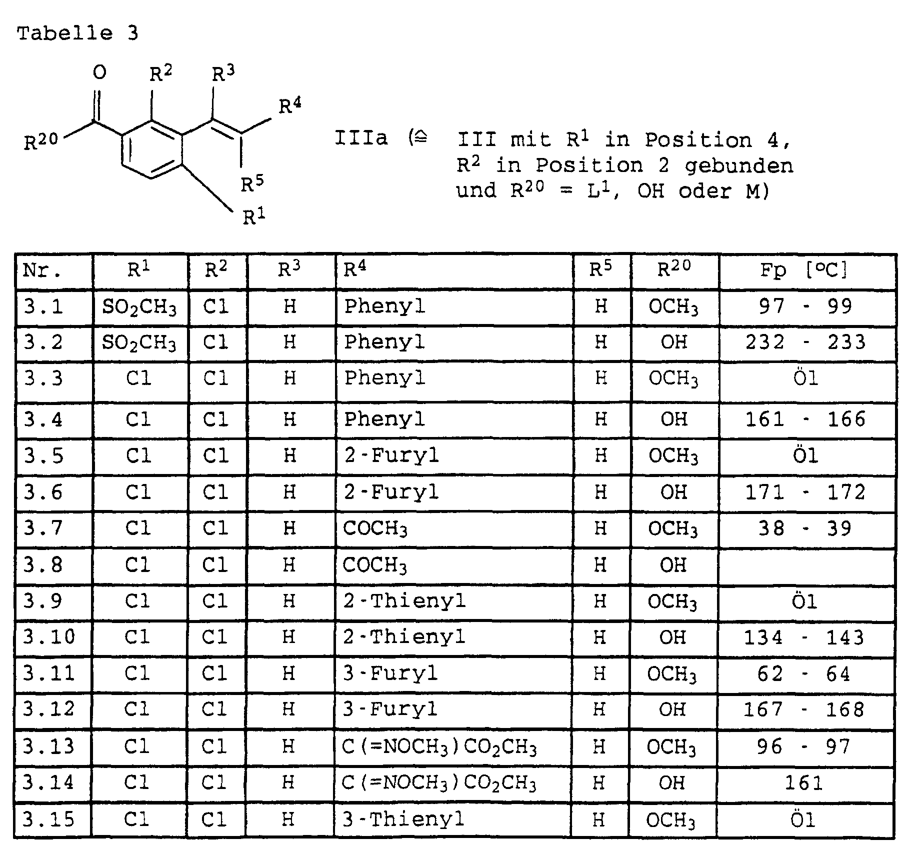

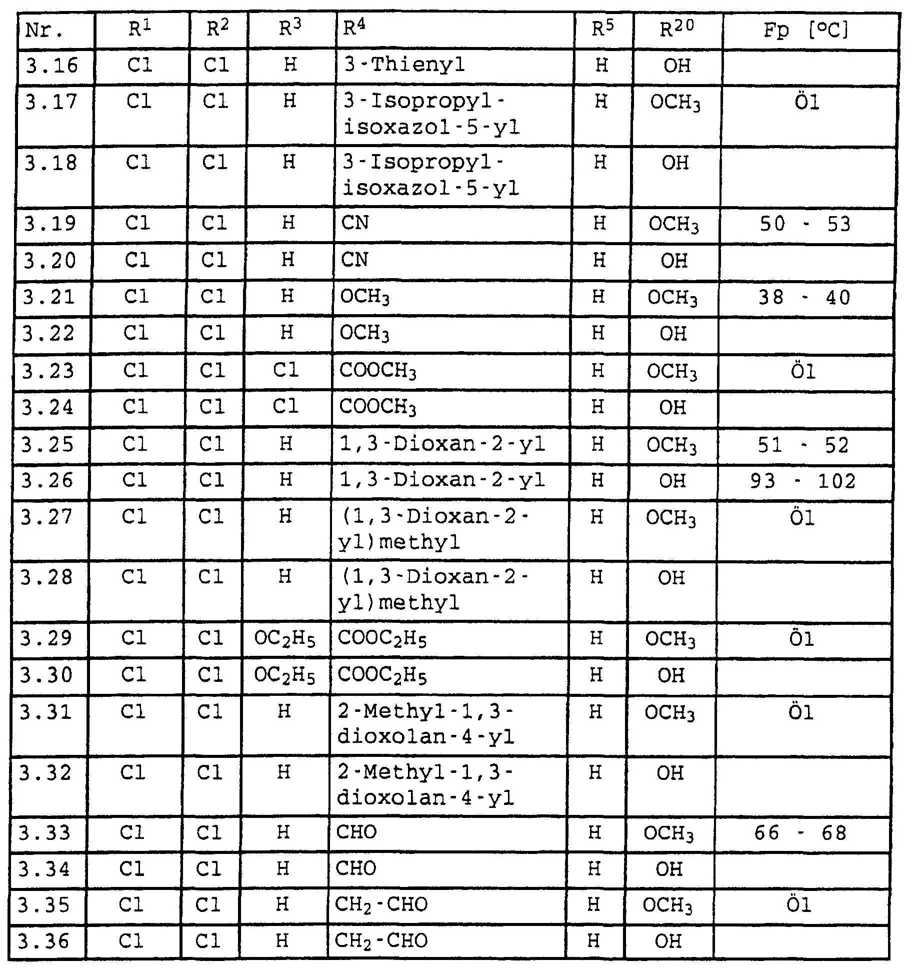

Die 2-(3-Alkenyl-benzoyl)-cyclohexan-1,3-dione der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmittel übliche Hilfsmittel.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die 2-(3-Alkenylbenzoyl)-cyclohexan-1,3-dione als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. 20 Gewichtsteile der Verbindung Nr. 2.2 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
II. 20 Gewichtsteile der Verbindung Nr. 2.3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
III. 20 Gewichtsteile des Wirkstoffs Nr. 2.4 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. 2.5 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile des Wirkstoffs Nr. 2.1 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.6 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. 2.11 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung Nr. 2.23 wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl) besteht. Man erhält ein stabiles Emulsionskonzentrat.

Die Applikation der Wirkstoffe der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy/Hetaryloxyalkansäure und deren Derivate, Benzoesäure und deren Derivate, Benzothiadiazinone, 2-(Aroyl/Hetaroyl)-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.)

### Anwendungsbeispiele

Die herbizide Wirkung der 2-(3-Alkenyl-benzoyl)-cyclohexan-1,3-dione der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,5 bzw. 0,25 kg/ha a. S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | | |
|---|---|---|
| Lateinischer Name | Englischer Name | Deutscher Name |
| Chenopodium album | lambsquarters (goosefoot) | Weißer Gänsefuß |
| Polygonum persicaria | ladysthumb | Flöhknöterich |
| Sinapis alba | white mustard | Weißer Senf |
| Solanum nigrum | black nightshade | Schwarzer Nachtschatten |

Oben genannte Unkräuter werden von Verbindung 2.1 im Nachauflauf bei Aufwandmengen von 0,5 bzw. 0,25 kg/ha a. S. sehr gut bekämpft.

## Patentansprüche

1. 2-(3-Alkenyl-benzoyl)-cyclohexan-1,3-dione der Formel I 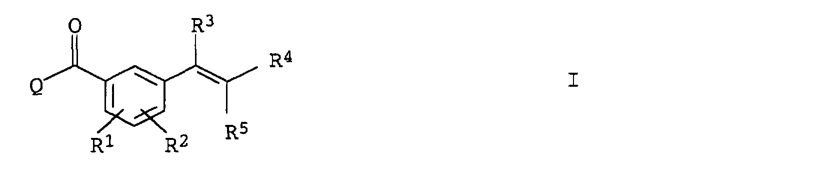 in der die Variablen folgende Bedeutung haben:
R¹, R² Wasserstoff, Nitro, Halogen, Cyano, Rhodano, C₁-C₆-Alkyl, C₁-C₆-Halogenalkyl, C₁-C₆-Alkoxy--C₁-C₆-alkyl, C₂-C₆-Alkenyl, C₂-C₆-Alkinyl, -OR⁶, -OCOR⁷, -OSO₂R⁷, -SH, -S(O)ₙR⁸, -SO₂OR⁶, -SO₂NR⁶R⁹, -NR⁹SO₂R⁷ oder -NR⁹COR⁷;
R³ Wasserstoff, Halogen, C₁-C₆-Alkyl, C₁-C₆-Halogenalkyl, C₁-C₆-Alkoxy, C₂-C₆-Alkenyl, C₂-C₆-Alkinyl;
R⁴, R⁵ Wasserstoff, Nitro, Halogen, Cyano, Rhodano, C₁-C₆-Alkyl, C₁-C₆-Halogenalkyl, C₃-C₆-Cycloalkyl, C₂-C₆-Alkenyl, C₄-C₆-Cycloalkenyl, C₂-C₆-Alkinyl, C₁-C₆-Alkoxy, C₁-C₆-Alkylthio, C₁-C₆-Halogenalkoxy, -COR¹⁰, -CO₂R¹⁰, -COSR¹⁰, -CONR¹⁰R¹¹, -C(R¹²)=NR¹³, -PO(OR¹⁰) (OR¹¹), C₁-C₄-Alkyl, das einen Rest aus folgender Gruppe trägt: Hydroxy, -COR¹⁰, -CO₂R¹⁰, -COSR¹⁰, -CONR¹⁰R¹¹ oder -C(R¹²)=NR¹³; Heterocyclyl, Heterocyclyl-C₁-C₄-alkyl, Phenyl, Phenyl-C₁-C₄-alkyl, Hetaryl, Hetaryl-C₁-C₄-alkyl, wobei die sechs letztgenannten Reste substituiert sein können;
oder
R⁴ und R⁵ bilden gemeinsam eine C₂-C₆-Alkandiylkette, die ein- bis vierfach durch C₁-C₄-Alkyl substituiert sein kann und/oder durch Sauerstoff oder Schwefel oder einen gegebenenfalls C₁-C₄-Alkyl substituierten Stickstoff unterbrochen sein kann;
n 0, 1 oder 2;
R⁶ Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
R⁷ C₁-C₆-Alkyl oder C₁-C₆-Halogenalkyl;
R⁸ C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
R⁹ Wasserstoff oder C₁-C₆-Alkyl;
R¹⁰ Wasserstoff, C₁-C₆-Alkyl, C₃-C₆-Cycloalkyl, C₁-C₆-Halogenalkyl, C₃-C₆-Alkenyl, C₃-C₆-Alkinyl, Phenyl oder Benzyl, wobei die beiden letztgenannten Reste partiell oder vollständig halogeniert sein können und/oder einen bis drei Reste aus der folgenden Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
R¹¹ Wasserstoff, C₁-C₆-Alkyl, C₃-C₆-Alkenyl oder C₃-C₆-Alkinyl;
oder
R¹⁰ und R¹¹ bilden gemeinsame eine C₂-C₆-Alkandiylkette, die ein- bis vierfach durch C₁-C₄-Alkyl substituiert sein kann und/oder durch Sauerstoff oder Schwefel oder einen gegebenenfalls C₁-C₄-Alkyl substituierten Stickstoff unterbrochen sein kann;
R¹² Wasserstoff, C₁-C₆-Alkyl, C₁-C₆-Halogenalkyl, C₁-C₆-Alkoxy, C₁-C₆-Alkoxycarbonyl, C₃-C₆-Cycloalkyl, C₂-C₆-Alkenyl, C₂-C₆-Alkinyl, Phenyl oder Benzyl, wobei die beiden letztgenannten Reste partiell oder vollständig halogeniert sein können und/oder einen bis drei Reste aus der folgenden Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
R¹³ C₁-C₆-Alkyl, C₁-C₆-Halogenalkyl, C₃-C₆-Cycloalkyl, C₃-C₆-Alkenyl, C₃-C₆-Alkinyl, C₁-C₆-Alkoxy, C₁-C₆-Halogenalkoxy, C₃-C₆-Cycloalkyloxy, C₃-C₆-Alkenyloxy, C₃-C₆-Alkinyloxy, Phenyl, Benzyl oder Phenyl-C₁-C₄-alkoxy, wobei die drei letztgenannten Reste partiell oder vollständig halogeniert sein können und/oder einen bis drei Reste aus der folgenden Gruppe tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;
Q ein unsubstituierter oder substituierter in 2-Stellung verknüpfter Cyclohexan-1,3-dionring der Formel II 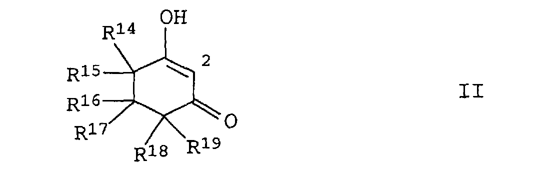 ist, wobei
R¹⁴, R¹⁵, R¹⁶, R¹⁷, R¹⁸ und R¹⁹ für Wasserstoff oder C₁-C₄-Alkyl stehen;
oder
die CR¹⁶R¹⁷-Einheit durch C=O ersetzt sein kann;
sowie deren landwirtschaftlich brauchbaren Salze.

2. 2-(3-Alkenyl-benzoyl)-cyclohexan-1,3-dione der Formel I nach Anspruch 1, in der
R⁴, R⁵ Wasserstoff, Nitro, Halogen, Cyano, Rhodano, C₁-C₆-Alkyl, C₁-C₆-Halogenalkyl, C₃-C₆-Cycloalkyl, C₂-C₆-Alkenyl, C₄-C₆-Cycloalkenyl, C₂-C₆-Alkinyl, C₁-C₆-Alkoxy, C₁-C₆-Alkylthio, C₁-C₆-Halogenalkoxy, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-Alkyl, das einen Rest aus folgender Gruppe trägt: -COR¹⁰, -CO₂R¹⁰, -COSR¹⁰, -CONR¹⁰R¹¹ oder -C(R¹²)=NR¹³; Heterocyclyl, Heterocyclyl-C₁-C₄-alkyl, Phenyl, Phenyl-C₁-C₄-alkyl, Hetaryl, Hetaryl-C₁-C₄-alkyl, wobei die sechs letztgenannten Reste substituiert sein können;
oder
R⁴ und R⁵ bilden gemeinsam eine C₂-C₆-Alkandiylkette, die ein- bis vierfach durch C₁-C₄-Alkyl substituiert sein kann und/oder durch Sauerstoff oder Schwefel oder einen gegebenenfalls C₁-C₄-Alkyl substituierten Stickstoff unterbrochen sein kann;
R¹³ C₁-C₆-Alkyl, C₁-C₆-Halogenalkyl, C₃-C₆-Cycloalkyl, C₃-C₆-Alkenyl, C₃-C₆-Alkinyl, C₁-C₆-Alkoxy, C₁-C₆-Halogenalkoxy, C₃-C₆-Cycloalkoxy, C₃-C₆-Alkenyloxy, C₃-C₆-Alkinyloxy, Phenyl, Benzyl oder Benzyloxy, wobei die drei letztgenannten Reste partiell oder vollständig halogeniert sein können und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkylcarbonyl oder C₁-C₄-Alkoxycarbonyl;

3. 2-(3-Alkenyl-benzoyl)-cyclohexan-1,3-dione der Formel I nach den Ansprüchen 1 oder 2, in der
R¹ Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁶ oder -S(O)ₙR⁸ bedeutet;
R² für Wasserstoff oder einen wie voranstehend unter R¹ genannten Rest steht.

4. 2-(3-Alkenyl-benzoyl)-cyclohexan-1,3-dione der Formel I nach Anspruch 1 bis 3, in der
R⁴ Wasserstoff, Nitro, Halogen, Cyano, Rhodano, C₁-C₆-Alkyl, C₁-C₆-Halogenalkyl, C₃-C₆-Cycloalkyl, C₂-C₆-Alkenyl, C₄-C₆-Cycloalkenyl, C₂-C₆-Alkinyl, C₁-C₆-Alkoxy, C₁-C₆-Alkylthio, C₁-C₆-Halogenalkoxy, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-Alkyl, das einen Rest aus folgender Gruppe trägt: -COR¹⁰, -CO₂R¹⁰, -COSR¹⁰, -CONR¹⁰R¹¹ oder -C(R¹²)=NR¹³; Heterocyclyl, Heterocyclyl-C₁-C₄-alkyl, Phenyl, Phenyl-C₁-C₄-alkyl, Hetaryl, Hetaryl-C₁-C₄-alkyl, wobei die sechs letztgenannten Reste substituiert sein können;
R⁵ Wasserstoff, Halogen, Cyano, Rhodano, C₁-C₆-Alkyl, C₁-C₆-Halogenalkyl, -COR¹⁰, -CO₂R¹⁰, -COSR¹⁰, -CONR¹⁰R¹¹ oder -PO(OR¹⁰) (OR¹¹);
oder
R⁴ und R⁵ gemeinsam eine C₂-C₆-Alkandiylkette, die ein- bis vierfach durch C₁-C₄-Alkyl substituiert sein kann und/oder durch Sauerstoff oder Schwefel oder einen gegebenenfalls C₁-C₄-Alkyl substiuierten Stickstoff unterbrochen sein kann;
bedeuten.

5. 2-(3-Alkenyl-benzoyl)-cyclohexan-1,3-dione der Formel Ia 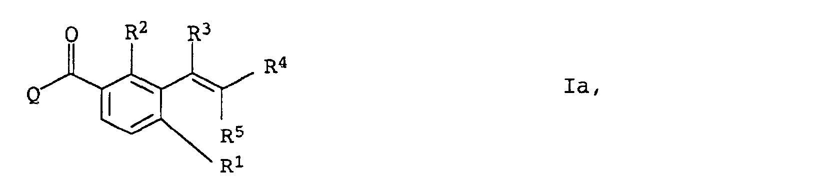 in der die Variablen R¹ bis R⁵ und Q die unter den Ansprüchen 1 bis 4 genannte Bedeutung haben.

6. Verfahren zur Herstellung von 2-(3-Alkenyl-benzoyl)-cyclohexan-1,3-dionen der Formel I gemäß den Ansprüchen 1 bis 5,
**dadurch gekennzeichnet, daß** man ein gegebenenfalls substituiertes Cyclohexan-1,3-dion Q mit einer aktivierten Carbonsäure IIIα oder mit einer Carbonsäure IIIβ, 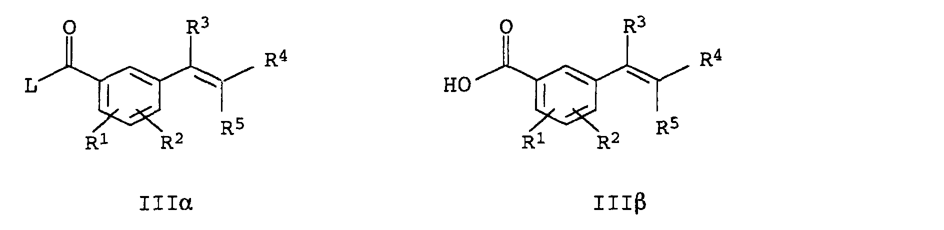 wobei die Variablen R¹ bis R⁵ die unter Anspruch 1 genannte Bedeutung haben und L für eine nucleophil verdrängbare Abgangsgruppe steht, acyliert und das Acylierungsprodukt gegebenenfalls in Gegenwart eines Katalysators zu den Verbindungen I umlagert.

7. Mittel, enthaltend eine herbizid wirksame Menge mindestens einer Verbindung der Formel I oder eines landwirtschaftlich brauchbaren Salzes von I, gemäß den Ansprüchen 1 bis 5, und für die Formulierung von Pflanzenschutzmitteln übliche Hilfsmittel.

8. Verfahren zur Herstellung von herbizid wirksamen Mitteln gemäß Anspruch 7, **dadurch gekennzeichnet, daß** man eine herbizid wirksame Menge mindestens einer Verbindung der Formel I oder eines landwirtschaftlich brauchbaren Salzes von I gemäß den Ansprüchen 1 bis 5 und für die Formulierung von Pflanzenschutzmitteln übliche Hilfsmittel mischt.

9. Verfahren zur Bekämpfung von unerwünschtem Pflanzenwuchs, **dadurch gekennzeichnet, daß** man eine herbizid wirksame Menge mindestens einer Verbindung der Formel I oder eines landwirtschaftlich brauchbaren Salzes von I gemäß den Ansprüchen 1 bis 5 auf Pflanzen, deren Lebensraum und/oder auf Samen einwirken läßt.

10. Verwendung der Verbindungen der Formel I und deren landwirtschaftlich brauchbaren Salze gemäß den Ansprüchen 1 bis 5 als Herbizide.

## Claims

1. A 2-(3-alkenylbenzoyl)cyclohexane-1,3-dione of the formula I 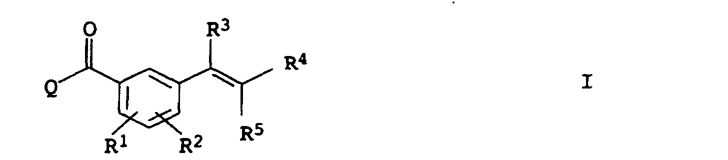 where the variables have the following meanings:
R¹, R² are hydrogen, nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁶, -OCOR⁷, -OSO₂R⁷, -SH, -S(O)ₙR⁸, -SO₂OR⁶, -SO₂NR⁶R⁹, -NR⁹SO₂R⁷ or -NR⁹COR⁷;
R³ is hydrogen, halogen, C₁-C₆-alkyl, C₁-C₆-haloalkyl, C₁-C₆-alkoxy, C₂-C₆-alkenyl, C₂-C₆-alkynyl;
R⁴, R⁵ are hydrogen, nitro, halogen, cyano, thiocyanato, C₁-C₆-alkyl, C₁-C₆-haloalkyl, C₃-C₆-cycloalkyl, C₂-C₆-alkenyl, C₄-C₆-cycloalkenyl, C₂-C₆-alkynyl, C₁-C₆-alkoxy, C₁-C₆-alkylthio, C₁-C₆-haloalkoxy, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-alkyl which has attached to it a radical from amongst the following group: hydroxyl, -COR¹⁰, -CO₂R¹⁰, -COSR¹⁰, -CONR¹⁰R¹¹ or -C(R¹²)=NR¹³; heterocyclyl,
heterocyclyl-C₁-C₄-alkyl, phenyl, phenyl-C₁-C₄-alkyl, hetaryl, hetaryl-C₁-C₄-alkyl, it being possible for the six last-mentioned radicals to be substituted;
or
R⁴ and R⁵ together form a C₂-C₆-alkanediyl chain which can be mono- to tetrasubstituted by C₁-C₄-alkyl and/or can be interrupted by oxygen or sulfur or by a nitrogen which is unsubstituted or substituted by C₁-C₄-alkyl;
n is 0, 1 or 2;
R⁶ is hydrogen, C₁-C₆-alkyl, C₁-C₆-haloalkyl, C₁-C₆-alkoxy-C₂-C₆-alkyl, C₃-C₆-alkenyl or C₃-C₆-alkynyl;
R⁷ is C₁-C₆-alkyl or C₁-C₆-haloalkyl;
R⁸ is C₁-C₆-alkyl, C₁-C₆-haloalkyl, C₁-C₆-alkoxy-C₂-C₆-alkyl, C₃-C₆-alkenyl or C₃-C₆-alkynyl;
R⁹ is hydrogen or C₁-C₆-alkyl;
R¹⁰ is hydrogen, C₁-C₆-alkyl, C₃-C₆-cycloalkyl, C₁-C₆-haloalkyl, C₃-C₆-alkenyl, C₃-C₆-alkynyl, phenyl or benzyl, it being possible for the two last-mentioned radicals to be partially or fully halogenated and/or to have attached to them one to three radicals from amongst the following group:
nitro, cyano, C₁-C₄-alkyl, C₁-C₄-haloalkyl, C₁-C₄-alkoxy, C₁-C₄-haloalkoxy, C₁-C₄-alkylcarbonyl or C₁-C₄-alkoxycarbonyl;
R¹¹ is hydrogen, C₁-C₆-alkyl, C₃-C₆-alkenyl or C₃-C₆-alkynyl;
or
R¹⁰ and R¹¹ together form a C₂-C₆-alkanediyl chain which can be mono- to tetrasubstituted by C₁-C₄-alkyl and/or can be interrupted by oxygen or sulfur or by a nitrogen which is unsubstituted or substituted by C₁-C₄-alkyl;
R¹² is hydrogen, C₁-C₆-alkyl, C₁-C₆-haloalkyl, C₁-C₆-alkoxy, C₁-C₆-alkoxycarbonyl, C₃-C₆-cycloalkyl, C₂-C₆-alkenyl, C₂-C₆-alkynyl, phenyl or benzyl, it being possible for the two last-mentioned radicals to be partially or fully halogenated and/or to have attached to them one to three radicals from amongst the following group: nitro, cyano, C₁-C₄-alkyl, C₁-C₄-haloalkyl, C₁-C₄-alkoxy, C₁-C₄-haloalkoxy, C₁-C₄-alkylcarbonyl or C₁-C₄-alkoxycarbonyl;
R¹³ is C₁-C₆-alkyl, C₁-C₆-haloalkyl, C₃-C₆-cycloalkyl, C₃-C₆-alkenyl, C₃-C₆-alkynyl, C₁-C₆-alkoxy, C₁-C₆-haloalkoxy, C₃-C₆-cycloalkyloxy, C₃-C₆-alkenyloxy, C₃-C₆-alkynyloxy, phenyl, benzyl or phenyl-C₁-C₄-alkoxy, it being possible for the three last-mentioned radicals to be partially or fully halogenated and/or to have attached to them one to three radicals from amongst the following group:
nitro, cyano, C₁-C₄-alkyl, C₁-C₄-haloalkyl, C₁-C₄-alkoxy, C₁-C₄-haloalkoxy, C₁-C₄-alkylcarbonyl or C₁-C₄-alkoxycarbonyl;
Q is an unsubstituted or substituted cyclohexane-1,3-dione ring of the formula II 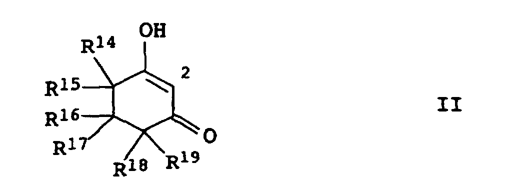 which is linked in the 2-position, where
R¹⁴, R¹⁵, R¹⁶, R¹⁷, R¹⁸ and R¹⁹ are hydrogen or C₁-C₄-alkyl;
the CR¹⁶R¹⁷ unit may be replaced by C=O;
or an agriculturally useful salt thereof.

2. A 2-(3-alkenylbenzoyl)cyclohexane-1,3-dione of the formula I as claimed in claim 1 where
R⁴, R⁵ are hydrogen, nitro, halogen, cyano, thiocyanato, C₁-C₆-alkyl, C₁-C₆-haloalkyl, C₃-C₆-cycloalkyl, C₂-C₆-alkenyl, C₄-C₆-cycloalkenyl, C₂-C₆-alkynyl, C₁-C₆-alkoxy, C₁-C₆-alkylthio, C₁-C₆-haloalkoxy, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-alkyl which has attached to it a radical from amongst the following group: -COR¹⁰, -CO₂R¹⁰, -COSR¹⁰, -CONR¹⁰R¹¹ or -C(R¹²)=NR¹³; heterocyclyl, heterocyclyl-C₁-C₄-alkyl, phenyl, phenyl-C₁-C₄-alkyl, hetaryl, hetaryl-C₁-C₄-alkyl, it being possible for the six last-mentioned radicals to be substituted;
or
R⁴ and R⁵ together form a C₂-C₆-alkanediyl chain which can be mono- to tetrasubstituted by C₁-C₄-alkyl and/or interrupted by oxygen or sulfur or by a nitrogen which is unsubstituted or substituted by C₁-C₄-alkyl; and
R¹³ is C₁-C₆-alkyl, C₁-C₆-haloalkyl, C₃-C₆-cycloalkyl, C₃-C₆-alkenyl, C₃-C₆-alkynyl, C₁-C₆-alkoxy, C₁-C₆-haloalkoxy, C₃-C₆-cycloalkoxy, C₃-C₆-alkenyloxy, C₃-C₆-alkynyloxy, phenyl, benzyl or benzyloxy, it being possible for the three last-mentioned radicals to be partially or fully halogenated and/or to have attached to them one to three radicals from amongst the following group: nitro, cyano, C₁-C₄-alkyl, C₁-C₄-haloalkyl, C₁-C₄-alkoxy, C₁-C₄-haloalkoxy, C₁-C₄-alkylcarbonyl or C₁-C₄-alkoxycarbonyl.

3. A 2-(3-alkenylbenzoyl)cyclohexane-1,3-dione of the formula I as claimed in either of claims 1 and 2 where
R¹ is nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁶ or -S(O)ₙR⁸;
R² is hydrogen or a radical as mentioned above under R¹.

4. A 2-(3-alkenylbenzoyl)cyclohexane-1,3-dione of the formula I as claimed in any of claims 1 to 3 where
R⁴ is hydrogen, nitro, halogen, cyano, thiocyanato, C₁-C₆-alkyl, C₁-C₆-haloalkyl, C₃-C₆-cycloalkyl, C₂-C₆-alkenyl, C₄-C₆-cycloalkenyl, C₂-C₆-alkynyl, C₁-C₆-alkoxy, C₁-C₆-alkylthio, C₁-C₆-haloalkoxy, -COR¹⁰, -CO₂R¹⁰, -COSR¹⁰, -CONR¹⁰R¹¹, -C(R¹²)=NR¹³, -PO(OR¹⁰)(OR¹¹), C₁-C₄-alkyl which has attached to it a radical from amongst the following group: -COR¹⁰, -CO₂R¹⁰, -COSR¹⁰, -CONR¹⁰R¹¹ or -C(R¹²)=NR¹³; heterocyclyl, heterocyclyl-C₁-C₄-alkyl, phenyl, phenyl-C₁-C₄-alkyl, hetaryl, hetaryl-C₁-C₄-alkyl, it being possible for the six last-mentioned radicals to be substituted;
R⁵ is hydrogen, halogen, cyano, thiocyanato, C₁-C₆-alkyl, C₁-C₆-haloalkyl, -COR¹⁰, -CO₂R¹⁰, -COSR¹⁰, -CONR¹⁰R¹¹ or -PO(OR¹⁰)(OR¹¹);
or
R⁴ and R⁵ together are a C₂-C₆-alkanediyl chain which can be mono- to tetrasubstituted by C₁-C₄-alkyl and/or can be interrupted by oxygen or sulfur or by a nitrogen which is unsubstituted or substituted by C₁-C₄-alkyl.

5. A 2-(3-alkenylbenzoyl)cyclohexane-1,3-dione of the formula Ia 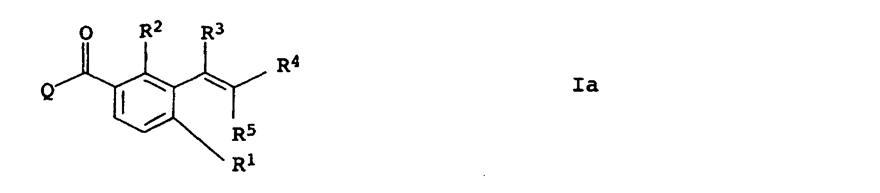 where the variables R¹ to R⁵ and Q have the meanings mentioned in claims 1 to 4.

6. A process for the preparation of a 2-(3-alkenylbenzoyl)cyclohexane-1,3-dione of the formula I as claimed in any of claims 1 to 5, which comprises acylating an unsubstituted or substituted cyclohexane-1,3-dione Q with an activated carboxylic acid IIIα or with a carboxylic acid IIIβ, 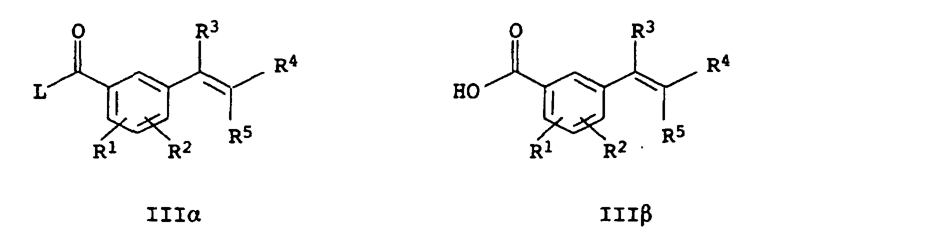 where the variables R¹ to R⁵ have the meanings mentioned in claim 1 and L is a nucleophilically displaceable leaving group, and, if desired, subjecting the acylation product to a rearrangement reaction in the presence of a catalyst to give the compounds I.

7. A composition comprising a herbicidally active amount of at least one compound of the formula I or of an agriculturally useful salt of I as claimed in any of claims 1 to 5 and auxiliaries conventionally used for the formulation of crop protection products.

8. A process for the preparation of a herbicidally active composition as claimed in claim 7, which comprises mixing a herbicidally active amount of at least one compound of the formula I or of an agriculturally useful salt of I as claimed in any of claims 1 to 5 and auxiliaries conventionally used for the formulation of crop protection products.

9. A method of controlling undesired vegetation, which comprises allowing a herbicidally active amount of at least one compound of the formula I or of an agriculturally useful salt of I as claimed in any of claims 1 to 5 to act on plants, their environment and/or on seeds.

10. The use of a compound of the formula I or an agriculturally useful salt thereof as claimed in any of claims 1 to 5 as herbicide.

## Revendications

1. 2-(3-alcényl-benzoyl)-cyclohexane-1,3-diones de formule I 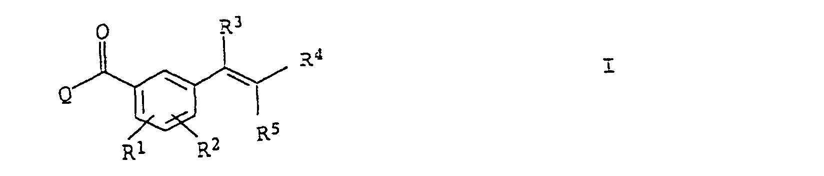 où les variables ont la signification suivante:
R¹, R² hydrogène, nitro, halogène, cyano, rhodano, alkyle en C₁-C₆, halogénoalkyle en C₁-C₆, alcoxy(C₁-C₆)alkyle(C₁-C₆), alcényle en C₂-C₆, alcynyle en C₂-C₆, -OR⁶, -OCOR⁷, -OSO₂R⁷, -SH, -S(O)ₙR⁸, -SO₂OR⁶, -SO₂NR⁶R⁹, -NR⁹SO₂R⁷ ou -NR⁹COR⁷;
R³ hydrogène, halogène, alkyle en C₁-C₆, halogénoalkyle en C₁-C₆, alcoxy en C₁-C₆, alcényle en C₂-C₆, alcynyle en C₂-C₆;
R⁴, R⁵ hydrogène, nitro, halogène, cyano, rhodano, alkyle en C₁-C₆, halogénoalkyle en C₁-C₆, cycloalkyle en C₃-C₆, alcényle en C₂-C₆, cycloalcényle en C₄-C₆, alcynyle en C₂-C₆, alcoxy en C₁-C₆, alkylthio en C₁-C₆, halogénoalcoxy en C₁-C₆, -COR¹⁰, -CO₂R¹⁰, -COSR¹⁰, -CONR¹⁰R¹¹, -C(R¹²)=NR¹³, -PO(OR¹⁰)(OR¹¹), alkyle en C₁-C₄ qui porte un reste choisi dans le groupe suivant: hydroxy, -COR¹⁰, -CO₂R¹⁰, -COSR¹⁰, -CONR¹⁰R¹¹ ou -C(R¹²)=NR¹³; hétérocyclyle, hétérocyclylalkyle(C₁-C₄), phényle, phényl-alkyle(C₁-C₄), hétaryle, hétaryl-alkyle(C₁-C₄), tandis que les six restes mentionnés en dernier peuvent être substitués;
ou
R⁴ et R⁵ forment ensemble une chaîne alcanediyle en C₂-C₆, qui peut être substituée une à quatre fois par un groupe alkyle en C₁-C₄ et/ou peut être interrompue par de l'oxygène ou du soufre ou un atome d'azote éventuellement substitué par un alkyle en C₁-C₄;
n 0, 1 ou 2;
R⁶ hydrogène, alkyle en C₁-C₆, halogénoalkyle en C₁-C₆, alcoxy(C₁-C₆)-alkyle(C₂-C₆), alcényle en C₃-C₆ ou alcynyle en C₃-C₆;
R⁷ alkyle en C₁-C₆ ou halogénoalkyle en C₁-C₆;
R⁸ alkyle en C₁-C₆, halogénoalkyle en C₁-C₆, alcoxy(C₁-C₆)-alkyle(C₂-C₆), alcényle en C₃-C₆ ou alcynyle en C₃-C₆;
R⁹ hydrogène ou alkyle en C₁-C₆;
R¹⁰ hydrogène, alkyle en C₁-C₆, cycloalkyle en C₃-C₆, halogénoalkyle en C₁-C₆, alcényle en C₃-C₆, alcynyle en C₃-C₆, phényle ou benzyle, tandis que les deux restes mentionnés en dernier peuvent être partiellement ou totalement halogénés et/ou peuvent porter un à trois restes choisis dans le groupe suivant: nitro, cyano, alkyle en C₁-C₄, halogénoalkyle en C₁-C₄, alcoxy en C₁-C₄, halogénoalcoxy en C₁-C₄, alkyl(C₁-C₄)-carbonyle ou alcoxy(C₁-C₄)-carbonyle;
R¹¹ hydrogène, alkyle en C₁-C₆, alcényle en C₃-C₆ ou alcynyle en C₃-C₆;
ou
R¹⁰ et R¹¹ forment ensemble une chaîne alcanediyle en C₂-C₆, qui peut être substituée une à quatre fois par un groupe alkyle en C₁-C₄ et/ou peut être interrompue par de l'oxygène ou du soufre ou un atome d'azote éventuellement substitué par un alkyle en C₁-C₄;
R¹² hydrogène, alkyle en C₁-C₆, halogénoalkyle en C₁-C₆, alcoxy en C₁-C₆, alcoxy(C₁-C₆)-carbonyle, cycloalkyle en C₃-C₆, alcényle en C₂-C₆, alcynyle en C₂-C₆, phényle ou benzyle, tandis que les deux restes mentionnés en dernier peuvent être partiellement ou totalement halogénés et/ou peuvent porter un à trois restes choisis dans le groupe suivant:
nitro, cyano, alkyle en C₁-C₄, halogénoalkyle en C₁-C₄, alcoxy en C₁-C₄, halogénoalcoxy en C₁-C₄, alkyl(C₁-C₄)-carbonyle ou alcoxy(C₁-C₄)-carbonyle;
R¹³ alkyle en C₁-C₆, halogénoalkyle en C₁-C₆, cycloalkyle en C₃-C₆, alcényle en C₃-C₆, alcynyle en C₃-C₆, alcoxy en C₁-C₆, halogénoalcoxy en C₁-C₆, cycloalkyloxy en C₃-C₆, alcényloxy en C₃-C₆, alcynyloxy en C₃-C₆, phényle, benzyle ou phénylalcoxy(C₁-C₄), tandis que les trois restes mentionnés en dernier peuvent être partiellement ou totalement halogénés et/ou peuvent porter un à trois restes choisis dans le groupe suivant:
nitro, cyano, alkyle en C₁-C₄, halogénoalkyle en C₁-C₄, alcoxy en C₁-C₄, halogénoalcoxy en C₁-C₄, alkyl(C₁-C₄)-carbonyle ou alcoxy(C₁-C₄)-carbonyle;
Q un cycle cyclohexane-1,3-dione lié sur la position 2, substitué ou non substitué, de formule II 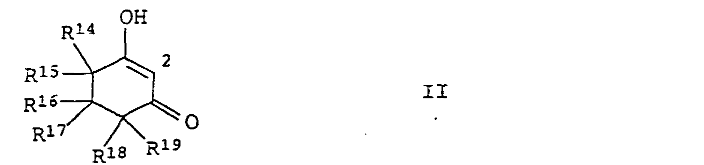 où
R¹⁴, R¹⁵, R¹⁶, R¹⁷, R¹⁸ et R¹⁹ désignent l'hydrogène ou un alkyle en C₁-C₄;
ou
l'unité CR¹⁶R¹⁷ peut être remplacée par C=O;
ainsi que leurs sels utilisables en agriculture.

2. 2-(3-alcényl-benzoyl)-cyclohexane-1,3-diones de formule I selon la revendication 1, dans laquelle
R⁴, R⁵ désigne l'hydrogène, nitro, halogène, cyano, rhodano, alkyle en C₁-C₆, halogénoalkyle en C₁-C₆, cycloalkyle en C₃-C₆, alcényle en C₂-C₆, cycloalcényle en C₄-C₆, alcynyle en C₂-C₆, alcoxy en C₁-C₆, alkylthio en C₁-C₆, halogénoalcoxy en C₁-C₆, -COR¹⁰, -CO₂R¹⁰, -COSR¹⁰, -CONR¹⁰R¹¹, -C(R¹²)=NR¹³, -PO(OR¹⁰)(OR¹¹), alkyle en C₁-C₄ qui porte un reste choisi dans le groupe suivant: -COR¹⁰, -CO₂R¹⁰, -COSR¹⁰, -CONR¹⁰R¹¹ ou -C(R¹²)=NR¹³ ; hétérocyclyle, hétérocyclyl-alkyle(C₁-C₄), phényle, phényl-alkyle(C₁-C₄), hétarylè, hétaryl-alkyle(C₁-C₄), tandis que les six restes mentionnés en dernier peuvent être substitués;
ou
R⁴ et R⁵ forment ensemble une chaîne alcanediyle en C₂-C₆, qui peut être substituée une à quatre fois par un groupe alkyle en C₁-C₄ et/ou peut être interrompue par de l'oxygène ou du soufre ou un atome d'azote éventuellement substitué par un alkyle en C₁-C₄;
R¹³ alkyle en C₁-C₆, halogénoalkyle en C₁-C₆, cycloalkyle en C₃-C₆, alcényle en C₃-C₆, alcynyle en C₃-C₆, alcoxy en C₁-C₆, halogénoalcoxy en C₁-C₆, cycloalkyloxy en C₃-C₆, alcényloxy en C₃-C₆, alcynyloxy en C₃-C₆, phényle, benzyle ou benzyloxy, tandis que les trois restes mentionnés en dernier peuvent être partiellement ou totalement halogénés et/ou peuvent porter un à trois restes choisis dans le groupe suivant: nitro, cyano, alkyle en C₁-C₄, halogénoalkyle en C₁-C₄, alcoxy en C₁-C₄, halogénoalcoxy en C₁-C₄, alkyl(C₁-C₄)-carbonyle ou alcoxy(C₁-C₄)-carbonyle.

3. 2-(3-alcényl-benzoyl)-cyclohexane-1,3-diones de formule I selon les revendications 1 ou 2, dans laquelle
R¹ désigne un groupe nitro, halogène, cyano, rhodano, alkyle en C₁-C₆, halogénoalkyle en C₁-C₆, alcoxy(C₁-C₆)alkyle(C₁-C₆), alcényle en C₂-C₆, alcynyle en C₂-C₆, -OR⁶ ou -S(O)ₙR⁸;
R² représente l'hydrogène ou un reste tel que mentionné plus haut sous R¹.

4. 2-(3-alcényl-benzoyl)-cyclohexane-1,3-diones de formule I selon la revendication 1 à 3, dans laquelle
R⁴ désigne l'hydrogène, nitro, halogène, cyano, rhodano, alkyle en C₁-C₆, halogénoalkyle en C₁-C₆, cycloalkyle en C₃-C₆, alcényle en C₂-C₆, cycloalcényle en C₄-C₆, alcynyle en C₂-C₆, alcoxy en C₁-C₆, alkylthio en C₁-C₆, halogénoalcoxy en C₁-C₆, -COR¹⁰, -CO₂R¹⁰, -COSR¹⁰, -CONR¹⁰R¹¹, -C(R¹²)=NR¹³, -PO(OR¹⁰)(OR¹¹), alkyle en C₁-C₄ qui porte un reste choisi dans le groupe suivant: -COR¹⁰, -CO₂R¹⁰, -COSR¹⁰, -CONR¹⁰R¹¹ ou -C(R¹²)=NR¹³; hétérocyclyle, hétérocyclyl-alkyle(C₁-C₄), phényle, phényl-alkyle(C₁-C₄), hétaryle, hétaryl-alkyle(C₁-C₄), tandis que les six restes mentionnés en dernier peuvent être substitués;
R⁵ désigne l'hydrogène, halogène, cyano, rhodano, alkyle en C₁-C₆, halogénoalkyle en C₁-C₆, -COR¹⁰, -CO₂R¹⁰, -COSR¹⁰, -CONR¹⁰R¹¹ ou -PO(OR¹⁰)(OR¹¹);
ou
R⁴ et R⁵ forment ensemble une chaîne alcanediyle en C₂-C₆, qui peut être substituée une à quatre fois par un groupe alkyle en C₁-C₄ et/ou peut être interrompue par de l'oxygène ou du soufre ou un atome d'azote éventuellement substitué par un alkyle en C₁-C₄.

5. 2-(3-alcényl-benzoyl)-cyclohexane-1,3-diones de formule Ia 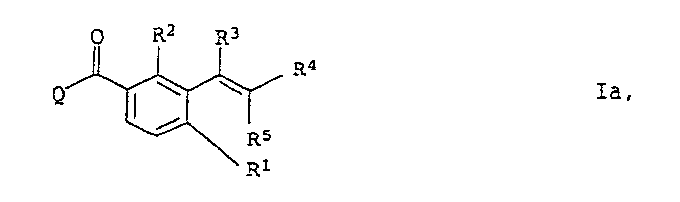 dans laquelle les variables R¹ à R⁵ et Q ont la signification indiquée dans les revendications 1 à 4.

6. Procédé pour la préparation de 2-(3-alcénylbenzoyl)-cyclohexane-1,3-diones de formule I selon les revendications 1 à 5, **caractérisé par le fait qu'**on acyle une cyclohexane-1,3-dione Q, éventuellement substituée, avec un acide carboxylique activé IIIα ou avec un acide carboxylique IIIβ, 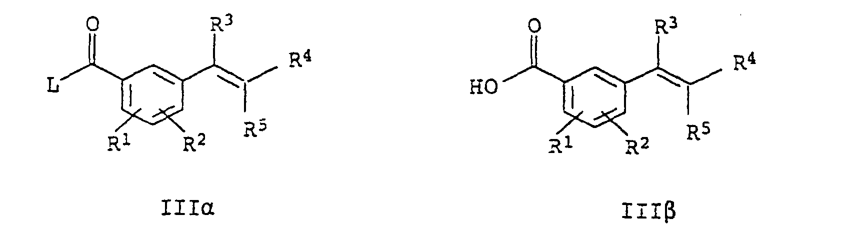 où les variables R¹ à R⁵ ont la signification indiquée dans la revendication 1 et L représente un groupe séparable pouvant être éliminé par voie nucléophile, et on convertit le produit d'acylation en les composés I, éventuellement en présence d'un catalyseur.

7. Produit, contenant une quantité à efficacité herbicide d'au moins un composé de formule I ou d'un sel de I utilisable en agriculture, selon les revendications 1 à 5, et des adjuvants classiques pour la formulation des produits phytosanitaires.

8. Procédé pour la préparation de produits à activité herbicide selon la revendication 7, **caractérisé par le fait qu'**on mélange une quantité à efficacité herbicide d'au moins un composé de formule I ou d'un sel de I utilisable en agriculture selon les revendications 1 à 5 et des adjuvants classiques pour la formulation des produits phytosanitaires.

9. Procédé pour la lutte contre la croissance des plantes non souhaitées, **caractérisé par le fait qu'**on fait agir une quantité à activité herbicide d'au moins un composé de formule I ou d'un sel de I utilisable en agriculture selon les revendications 1 à 5 sur des plantes, leur biotope et/ou des semences.

10. Utilisation des composés de formule I et de leurs sels utilisables en agriculture selon les revendications 1 à 5 comme herbicides.
